# EUROPEAN PATENT APPLICATION

(11) **EP 4 144 737 A1**
(43) Date of publication of application: **08.03.2023**
(21) Application number: 21797461.7
(22) Date of filing: 27.04.2021
(51) Int. Cl.: C07D 487/04, A61K 31/519

(54) **CRYSTAL FORMS OF SULFONAMIDE COMPOUND AND PREPARATION METHOD THEREFOR**

(30) Priority: 27.04.2020 CN 202010347086
(71) Applicant: Crystal Pharmatech Co., Ltd., Suzhou, Jiangsu 215123 (CN)
(72) Inventor: LU, Xia, Suzhou, Jiangsu 215123 (CN); ZHONG, Zhi, Suzhou, Jiangsu 215123 (CN); ZHANG, Xiaoyu, Suzhou, Jiangsu 215123 (CN)
(74) Representative: Grünecker Patent- und Rechtsanwälte PartG mbB
(86) International application number: PCT/CN2021/090151
(87) International publication number: WO 2021/218948

(57) **Abstract**

The present invention relates to crystalline forms of a sulfonamide compound and processes for preparing the same. The present invention provides five Crystalline Forms B, C, D, E, and F of the compound of formula (I), and processes for preparing the same and uses thereof. The prevent invention also provides processes for preparing the Crystalline Form A. Crystalline Forms B, C, D, E, and F of the compound of formula (I) provided by the present invention have the advantages in at least one of solubility, melting point, stability, dissolution, hygroscopicity, adhesion, fluidity, biological effectiveness, processing performance, purification, formulation production, safety, and the like, which provides a new and better choice for the preparation of pharmaceutical formulations containing the compound of formula (I), and has a very important significance for the drug development.

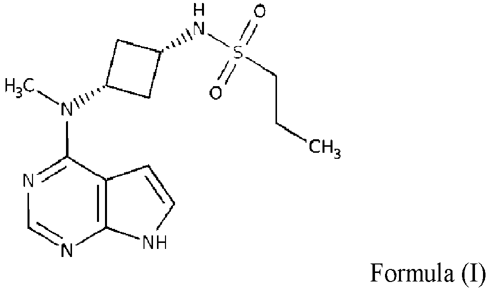

## Description

### Technical Field

The present invention relates to the field of chemical medicine, in particular to crystalline form of a sulfonamide compound and processes for preparing the same.

### Background Technology

JAK kinases are a family of cytoplasmic protein tyrosine kinases including JAK1, JAK2, JAK3 and TYK2. Each of the JAK kinases is selective for the receptors of certain cytokines, though multiple JAK kinases may be affected by particular cytokine or signaling pathways. As JAK1 pairs with JAK2, JAK3, and TYK2, a JAK1-selective inhibitor would be expected to inhibit many cytokines involved in inflammation and immune function while avoiding inhibition of the JAK2 homodimer regulating erythropoietin and thrombopoietin signaling. N-(((1S,3S)-3-(methyl(7H-pyrrolo[2,3-d]pyrimidin-4-yl)amino)cyclobutyl)propane-1-sulf onamide is a potent, highly selective JAK1 inhibitor, which is effective for autoimmune diseases, and has made certain progress in the treatment of atopic dermatitis. Its structural formula is as follows:

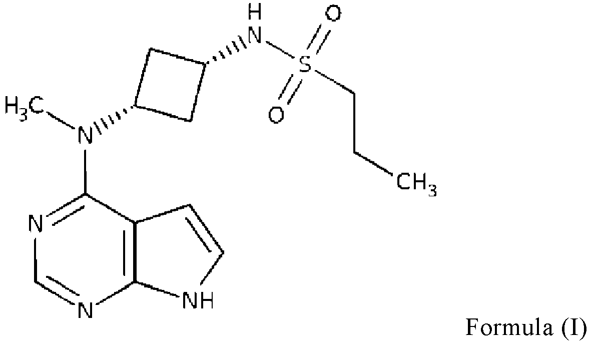

Patent US9035074B2 discloses the compound of formula (I) and its synthesis. The disclosed synthesis method requires using a mixed solution of dichloromethane and methanol and purifying with a chromatographic column, and has a yield of 78% only. The above-mentioned preparation process has a cumbersome procedure with a low yield and can produce toxic and harmful waste liquid, therefore a simpler, safer and higher-yield preparation process needs to be developed.

WO2020/008391A discloses a crystalline form of the compound of formula (I) (hereinafter, sometimes referred to as "Crystalline Form A"), and said crystalline form has an X-ray powder diffraction pattern comprising characteristic peaks at the 2θ values of 13.0°, 14.8°, and 23.3°±0.2°.

In addition, different crystalline forms of the same drug have significant differences in solubility, melting point, density, stability, and the like, thus affecting stability, homogeneity, bioavailability, efficacy and safety of the drug to greater or lesser degrees. Therefore, it is one of the important research contents that cannot be ignored to carry out comprehensive and systematic polymorph screening in drug research and development, and select the most suitable crystalline form for development. Based on this, it is necessary to screen the polymorphic form of compound (I) to provide more and better choices for the subsequent development of the drug.

### Summary of the Invention

The present invention provides five Crystalline Forms B, C, D, E, and F of the compound of formula (I), and processes for preparing the same and uses thereof. The prevent invention also provides processes for preparing the Crystalline Form A.
1. E-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form E, which is characterized in that, by using the Cu-Kα radiation, Crystalline Form E has an X-ray powder diffraction pattern comprising characteristic peaks at the 2θ values of 12.8°±0.2°, 15.3°±0.2°, and 21.6°±0.2°, 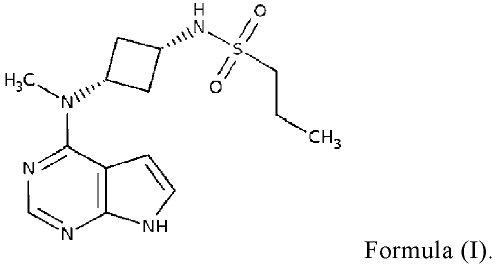
2. The process for preparing Crystalline Form E according to the above-mentioned item 1, which is characterized in that said process comprises:
   step (1): heating the compound of formula (I) to 200°C-220°C,
   step (2): then cooling to 0°C-5°C,
   step (3): reheating to 140°C-160°C to obtain Crystalline Form E.
3. F-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form F, which is characterized in that, by using the Cu-Kα radiation, Crystalline Form F has an X-ray powder diffraction pattern comprising characteristic peaks at the 2θ values of 10.9°±0.2°, 14.8°±0.2°, and 20.6°±0.2°, 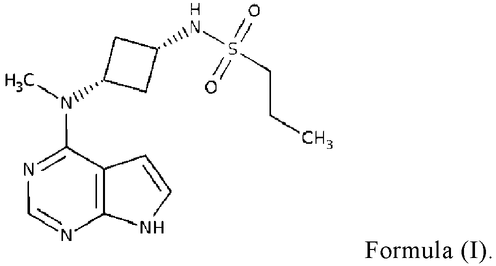
4. A process for preparing Crystalline Form F according to the above-mentioned item 3, which is characterized in that,
   a solid of the compound of formula (I) is heated, and then cooled to obtain Crystalline Form F.
5. C-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form C, which is characterized in that, by using the Cu-Kα radiation, Crystalline Form C has an X-ray powder diffraction pattern comprising characteristic peaks at the 2θ values of 5.6°±0.2°, 10.1°±0.2°, and 15.3°±0.2°, 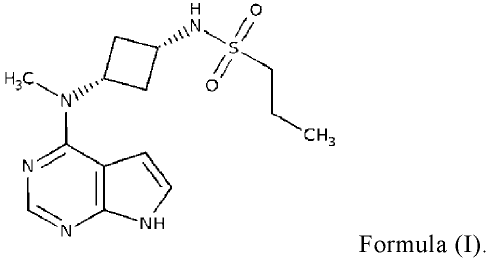
6. The process for preparing Crystalline Form C according to the above-mentioned item 5, which is characterized in that,
   the compound of formula (I) is dissolved in methanol/dichloromethane, the resulting system is placed under an atmosphere of methyl tert-butyl ether to perform the liquid vapor diffusion, then volatilized, and separated to obtain Crystalline Form C.
7. D-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form D, which is characterized in that, by using the Cu-Kα radiation, Crystalline Form D has an X-ray powder diffraction pattern comprising characteristic peaks at the 2θ values of 7.1°±0.2°, 14.2°±0.2°, and 15.1°±0.2°, 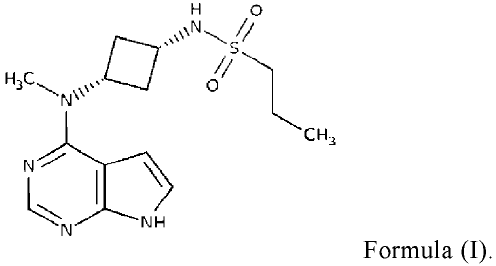
8. The process for preparing Crystalline Form D according to the above-mentioned item 7, which is characterized in that,
   the compound of formula (I) is dissolved in dimethyl sulfoxide, added to isopropyl acetate, and then transferred to a temperature between -25°C and -10°C to obtain Crystalline Form D.
9. B-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form B, which is characterized in that, by using the Cu-Kα radiation, Crystalline Form B has an X-ray powder diffraction pattern comprising characteristic peaks at the 2θ values of 11.5°±0.2°, 15.7°±0.2°, and 18.9°±0.2°, 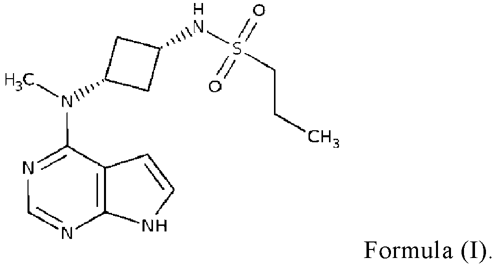
10. The process for preparing Crystalline Form B according to the above-mentioned item 9, which is characterized in that,
   the compound of formula (I) is added to toluene/dimethyl sulfoxide, and the suspension system is stirred to obtain Crystalline Form B.
11. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is characterized in that, 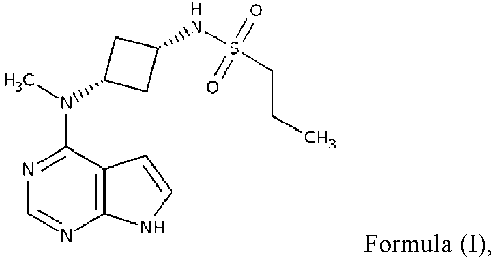 the compound of formula (I) is dissolved in a positive solvent, and after filtering, an anti-solvent is added dropwise thereto to obtain Crystalline Form A.
12. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is characterized in that, 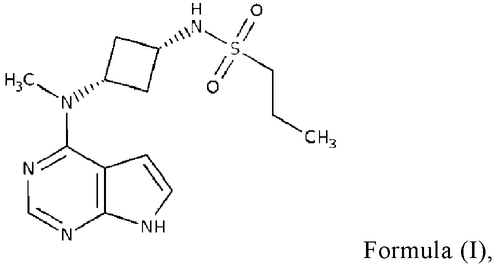 the compound of formula (I) is dissolved in a positive solvent, and after filtering, quickly added to an anti-solvent to obtain Crystalline Form A.
13. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is characterized in that, 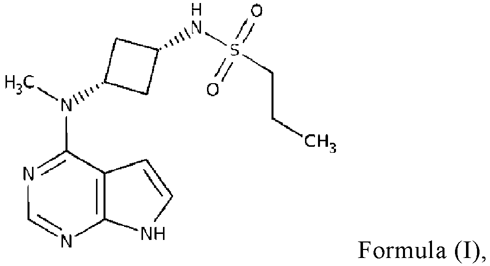 the compound of formula (I) is dissolved in at least one of methanol, tetrahydrofuran, 1,4-dioxane, trifluoroethanol, acetic acid, acetonitrile/pure water, methanol/dichloromethane, 40% aqueous dimethylamine solution and tetrahydrofuran/water, and after filtering, the solvent is volatilized until a solid precipitates to obtain Crystalline Form A.
14. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is characterized in that, 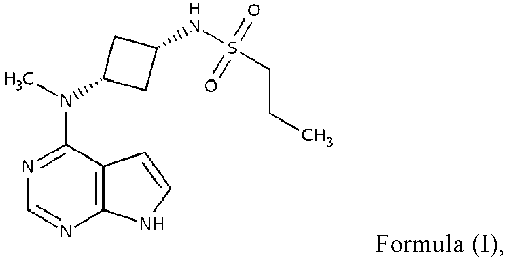 the compound of formula (I) is dissolved in an organic solvent or a mixed solvent system, filtered after reaching a dissolution equilibrium under a high temperature condition, and slowly cooled until a solid precipitates to obtain Crystalline Form A.
15. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is characterized in that, 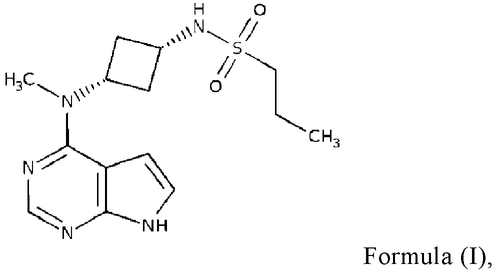 An excessive amount of the compound of formula (I) is added to an organic solvent or a mixed solvent system, and the suspension system is stirred to obtain Crystalline Form A.
16. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is characterized in that, 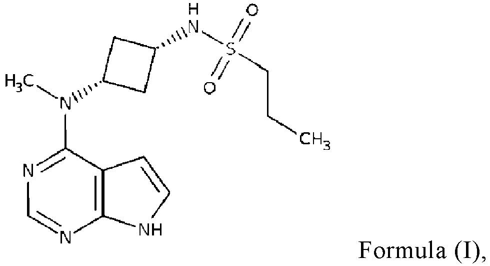 the compound of formula (I) is dissolved in a positive solvent, and placed under an anti-solvent atmosphere to perform the liquid vapor diffusion, and then the resulting system is volatilized and separated to obtain Crystalline Form A.
17. A pharmaceutical composition, containing the crystal according to any one of the above-mentioned items 1, 3, 5, 7 and 9 and a pharmaceutically acceptable carrier.
18. A pharmaceutical composition having JAK1 inhibitory activity, containing the crystal according to any one of the above-mentioned items 1, 3, 5, 7 and 9 as an active component.
19. A preventing or treating drug for atopic dermatitis, lupus vulgavis and/or plaque psoriasis, which contains the crystal according to any one of the above-mentioned items 1, 3, 5, 7 and 9 as an active component.
20. A process for preparing Crystalline Form F according to the above-mentioned item 3, which is characterized in that the compound of formula (I) is added to methanol/dichloromethane, and after filtering, the filtrate is subjected to the liquid vapor diffusion with methyl tert-butyl ether to obtain Crystalline Form F.

Compared with the prior art, Crystalline Forms B, C, D, E, and F of the compound of formula (I) provided by the present invention have the advantages in at least one of solubility, melting point, stability, dissolution, hygroscopicity, adhesion, fluidity, biological effectiveness, processing performance, purification, formulation production, safety, and the like, which provides a new and better choice for the preparation of pharmaceutical formulations containing the compound of formula (I), and has a very important significance for the drug development.

### Brief Description of the Drawings

Figure 1: XRPD pattern of Crystalline Form A
Figure 2: TGA diagram of Crystalline Form A
Figure 3: DSC chart of Crystalline Form A
Figure 4: ¹H NMR spectrum of Crystalline Form A
Figure 5: XRPD pattern of Crystalline Form B
Figure 6: XRPD pattern of Crystalline Form C
Figure 7: DSC chart of Crystalline Form C
Figure 8: XRPD pattern of Crystalline Form D
Figure 9: TGA diagram of Crystalline Form D
Figure 10: DSC chart of Crystalline Form D
Figure 11: XRPD pattern of Crystalline Form E
Figure 12: TGA diagram of Crystalline Form E
Figure 13: DSC chart of Crystalline Form E
Figure 14: XRPD pattern of Crystalline Form F of Example 30
Figure 15: XRPD pattern of Crystalline Form F of Example 31
Figure 16: Comparison of solubility of different crystalline forms in water
Figure 17: Comparison of solubility of different crystalline forms in FaSSIF
Figure 18: Comparison of solubility of different crystalline forms in FeSSIF
Figure 19: Comparison of XRPDs for stability testing of Crystalline Form E at 25°C/60% RH
Figure 20: Comparison of XRPDs for stability testing of Crystalline Form E at 40°C/75% RH
Figure 21: Comparison of XRPDs for stability testing of Crystalline Form F at 40°C/75% RH
Figure 22: Diagram of dynamic vapor sorption and desorption of Crystalline Form A
Figure 23: Diagram of dynamic vapor sorption and desorption of Crystalline Form E
Figure 24: TGA diagram of Crystalline Form F of Example 30
Figure 25: DSC chart of Crystalline Form F of Example 30

### Detailed Description of the Invention

### Crystalline Form B

B-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form B, is characterized in that, by using the Cu-Kα radiation, Crystalline Form B has an X-ray powder diffraction pattern comprising characteristic peaks at the 2θ values of 11.5°±0.2°, 15.7°±0.2°, and 18.9°±0.2°,

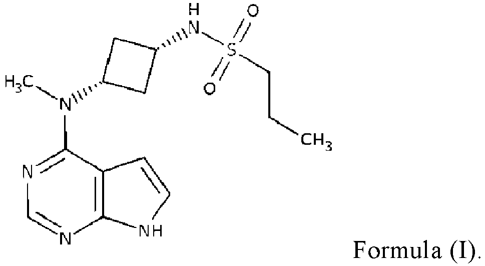

In an embodiment of the present invention, Crystalline Form B has an X-ray powder diffraction pattern comprising characteristic peak(s) at one, two or three 2θ values of 12.4°±0.2°, 21.3°±0.2°, 25.0°±0.2°.

In an embodiment of the present invention, Crystalline Form B has an X-ray powder diffraction pattern comprising characteristic peaks at the 2θ values of 12.4°±0.2°, 21.3°±0.2°, and 25.0°±0.2°.

In an embodiment of the present invention, Crystalline Form B has an X-ray powder diffraction pattern comprising characteristic peak(s) at one, two, or three 2θ values of 20.6°±0.2°, 22.6°±0.2°, 27.5°±0.2°.

In an embodiment of the present invention, Crystalline Form B has an X-ray powder diffraction pattern comprising characteristic peaks at the 2θ values of 20.6°±0.2°, 22.6°±0.2°, and 27.5°±0.2°.

In an embodiment of the present invention, Crystalline Form B has an X-ray powder diffraction pattern comprising characteristic peaks at any four, or five, or six, or seven, or eight, or nine 2θ values of 11.5°±0.2°, 12.4°±0.2°, 15.7°±0.2°, 18.9°±0.2°, 20.6°±0.2°, 21.3°±0.2°, 22.6°±0.2°, 25.0°±0.2°, 27.5°±0.2°.

In an embodiment of the present invention, Crystalline Form B has an X-ray powder diffraction pattern comprising characteristic peaks at the 2θ values of 11.5°±0.2°, 12.4°±0.2°, 15.7°±0.2°, 18.9°±0.2°, 20.6°±0.2°, 21.3°±0.2°, 22.6°±0.2°, 25.0°±0.2°, and 27.5°±0.2°.

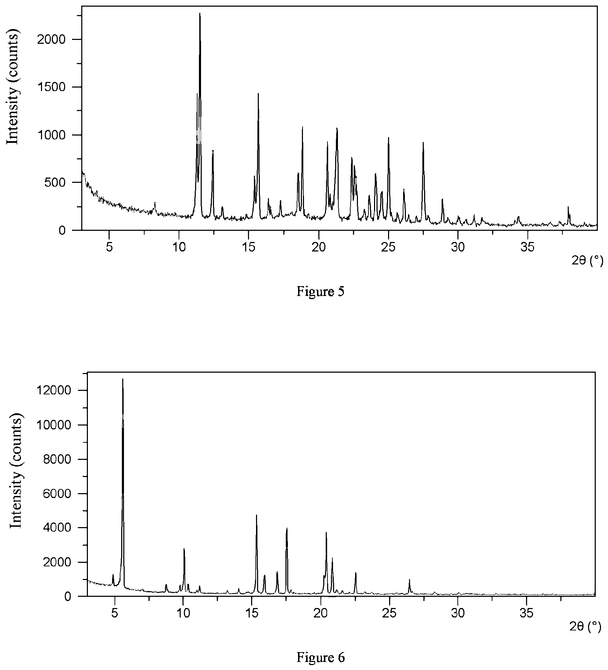

In an embodiment of the present invention, Crystalline Form B has an X-ray powder diffraction pattern as shown in Figure 5.

A process for preparing Crystalline Form B is characterized in that, the compound of formula (I) is added to toluene/dimethyl sulfoxide, and the suspension system is stirred to obtain Crystalline Form B.

According to the process for preparing Crystalline Form B, the ratio by volume of toluene/dimethyl sulfoxide is 2-4:0.5-2, for example, the ratio by volume of toluene/dimethyl sulfoxide is 3:1.

According to the process for preparing Crystalline Form B, the stirring is performed at 22°C to 28°C for 1 day to 21 days, for example, for 14 days.

### Crystalline Form C

C-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form C, is characterized in that, by using the Cu-Kα radiation, Crystalline Form C has an X-ray powder diffraction pattern comprising characteristic peaks at the 2θ values of 5.6°±0.2°, 10.1°±0.2°, and 15.3°±0.2°,

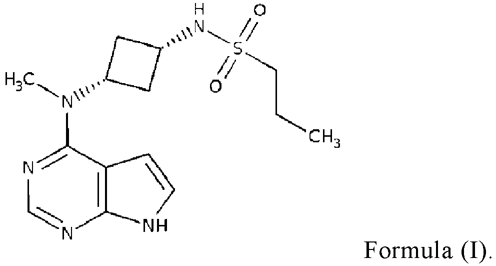

In an embodiment of the present invention, Crystalline Form C has an X-ray powder diffraction pattern comprising characteristic peak(s) at one, two, or three 2θ values of 15.9°±0.2°, 17.5°±0.2°, 20.4°±0.2°.

In an embodiment of the present invention, Crystalline Form C has an X-ray powder diffraction pattern comprising characteristic peaks at the 2θ values of 15.9°±0.2°, 17.5°±0.2°, and 20.4°±0.2°.

In an embodiment of the present invention, Crystalline Form C has an X-ray powder diffraction pattern comprising characteristic peak(s) at one, two, or three 2θ values of 16.8°±0.2°, 20.8°±0.2°, 22.5°±0.2°.

In an embodiment of the present invention, Crystalline Form C has an X-ray powder diffraction pattern comprising characteristic peaks at the 2θ values of 16.8°±0.2°, 20.8°±0.2°, and 22.5°±0.2°.

In an embodiment of the present invention, Crystalline Form C has an X-ray powder diffraction pattern comprising characteristic peaks at any four, or five, or six, or seven, or eight, or nine 2θ values of 5.6°±0.2°, 10.1°±0.2°, 15.3°±0.2°, 15.9°±0.2°, 16.8°±0.2°, 17.5°±0.2°, 20.4°±0.2°, 20.8°±0.2°, 22.5°±0.2°.

In an embodiment of the present invention, Crystalline Form C has an X-ray powder diffraction pattern comprising characteristic peaks at the 2θ values of 5.6°±0.2°, 10.1°±0.2°, 15.3°±0.2°, 15.9°±0.2°, 16.8°±0.2°, 17.5°±0.2°, 20.4°±0.2°, 20.8°±0.2°, and 22.5°±0.2°.

In an embodiment of the present invention, Crystalline Form C has an X-ray powder diffraction pattern as shown in Figure 6.

A process for preparing Crystalline Form C is characterized in that, the compound of formula (I) is dissolved in methanol/dichloromethane, the resulting system is placed under an atmosphere of methyl tert-butyl ether to perform the liquid vapor diffusion, then volatilized, and separated to obtain Crystalline Form C.

According to the process for preparing Crystalline Form C, the ratio by volume of methanol/dichloromethane is 0.5-2:0.5-2, for example, the ratio by volume of methanol/dichloromethane is 1:1.

The process for preparing Crystalline Form C is performed at 22°C to 28°C.

According to the process for preparing Crystalline Form C, the liquid vapor diffusion is performed for 2 days to 20 days, or 4 days to 16 days, for example, 12 days.

### Crystalline Form D

D-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form D, is characterized in that, by using the Cu-Kα radiation, Crystalline Form D has an X-ray powder diffraction pattern comprising characteristic peaks at the 2θ values of 7.1°±0.2°, 14.2°±0.2°, and 15.1°±0.2°,

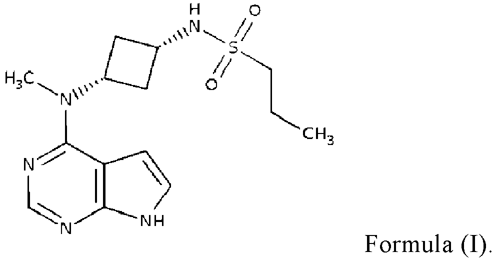

In an embodiment of the present invention, Crystalline Form D has an X-ray powder diffraction pattern comprising characteristic peak(s) at one, two, or three 2θ values of 11.3°±0.2°, 16.4°±0.2°, 22.2°±0.2°.

In an embodiment of the present invention, Crystalline Form D has an X-ray powder diffraction pattern comprising characteristic peaks at the 2θ values of 11.3°±0.2°, 16.4°±0.2°, and 22.2°±0.2°.

In an embodiment of the present invention, Crystalline Form D has an X-ray powder diffraction pattern comprising characteristic peak(s) at one, two, or three 2θ values of 17.2°±0.2°, 20.5°±0.2°, 22.8°±0.2°.

In an embodiment of the present invention, Crystalline Form D has an X-ray powder diffraction pattern comprising characteristic peaks at the 2θ values of 17.2°±0.2°, 20.5°±0.2°, and 22.8°±0.2°.

In an embodiment of the present invention, Crystalline Form D has an X-ray powder diffraction pattern comprising characteristic peaks at any four, or five, or six, or seven, or eight, or nine 2θ values of 7.1°±0.2°, 11.3°±0.2°, 14.2°±0.2°, 15.1°±0.2°, 16.4°±0.2°, 17.2°±0.2°, 20.5°±0.2°, 22.2°±0.2°, 22.8°±0.2°.

In an embodiment of the present invention, Crystalline Form D has an X-ray powder diffraction pattern comprising characteristic peaks at the 2θ values of 7.1°±0.2°, 11.3°±0.2°, 14.2°±0.2°, 15.1°±0.2°, 16.4°±0.2°, 17.2°±0.2°, 20.5°±0.2°, 22.2°±0.2°, and 22.8°±0.2°.

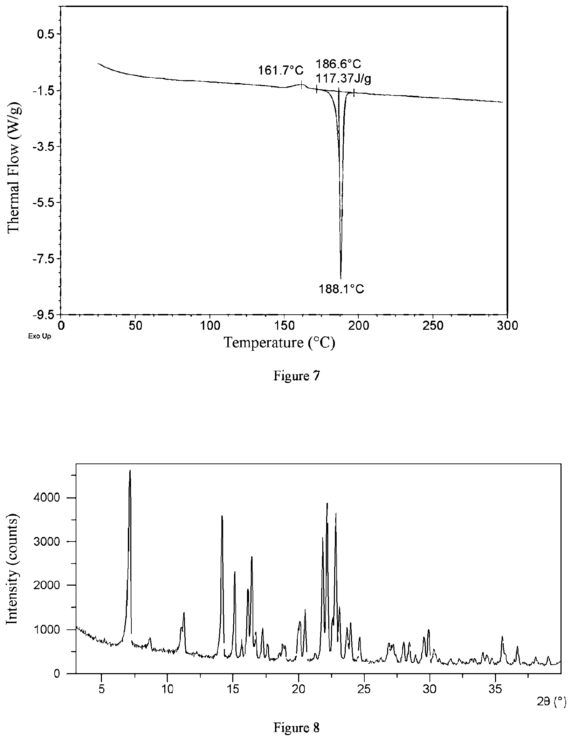

In an embodiment of the present invention, Crystalline Form D has an X-ray powder diffraction pattern as shown in Figure 8.

A process for preparing Crystalline Form D is characterized in that, the compound of formula (I) is dissolved in dimethyl sulfoxide, added to isopropyl acetate, and then transferred to a temperature between -25°C and -10°C to obtain Crystalline Form D. According to the process for preparing Crystalline Form D, the temperature for dissolving is 20°C to 30°C, and preferably transferred to a temperature between -25°C and -10°C, for example -20°C; the stirring is performed for 1 day to 10 days, for example 3 days.

### Crystalline Form E

E-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form E, is characterized in that, by using the Cu-Kα radiation, Crystalline Form E has an X-ray powder diffraction pattern comprising characteristic peaks at the 2θ values of 12.8°±0.2°, 15.3°±0.2°, and 21.6°±0.2°,

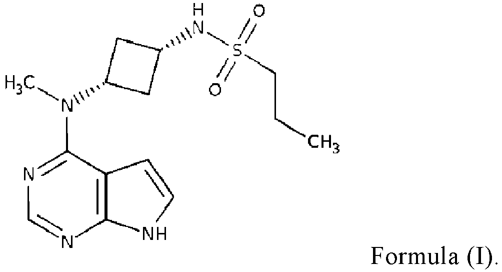

In an embodiment of the present invention, Crystalline Form E has an X-ray powder diffraction pattern comprising characteristic peak(s) at one, two, or three 2θ values of 14.7°±0.2°, 18.3°±0.2°, 19.4°±0.2°.

In an embodiment of the present invention, Crystalline Form E has an X-ray powder diffraction pattern comprising characteristic peaks at the 2θ values of 14.7°±0.2°, 18.3°±0.2°, and 19.4°±0.2°.

In an embodiment of the present invention, Crystalline Form E has an X-ray powder diffraction pattern comprising characteristic peak(s) at one, two, or three 2θ values of 16.1°±0.2°, 23.2°±0.2°, 23.8°±0.2°.

In an embodiment of the present invention, Crystalline Form E has an X-ray powder diffraction pattern comprising characteristic peaks at the 2θ values of 16.1°±0.2°, 23.2°±0.2°, and 23.8°±0.2°.

In an embodiment of the present invention, Crystalline Form E has an X-ray powder diffraction pattern comprising characteristic peaks at any four, or five, or six, or seven, or eight, or nine 2θ values of 12.8°±0.2°, 14.7°±0.2°, 15.3°±0.2°, 16.1°±0.2°, 18.3°±0.2°, 19.4°±0.2°, 21.6°±0.2°, 23.2°±0.2°, 23.8°±0.2°.

In an embodiment of the present invention, Crystalline Form E has an X-ray powder diffraction pattern comprising characteristic peaks at the 2θ values of 12.8°±0.2°, 15.3°±0.2°, 18.3°±0.2°, 19.4°±0.2°, 21.6°±0.2°, 23.8°±0.2°.

In an embodiment of the present invention, Crystalline Form E has an X-ray powder diffraction pattern comprising characteristic peaks at the 2θ values of 12.8°±0.2°, 14.7°±0.2°, 15.3°±0.2°, 18.3°±0.2°, 19.4°±0.2°, 21.6°±0.2°.

In an embodiment of the present invention, Crystalline Form E has an X-ray powder diffraction pattern comprising characteristic peaks at the 2θ values of 12.8°±0.2°, 14.7°±0.2°, 15.3°±0.2°, 16.1°±0.2°, 18.3°±0.2°, 19.4°±0.2°, 21.6°±0.2°, 23.2°±0.2°, and 23.8°±0.2°.

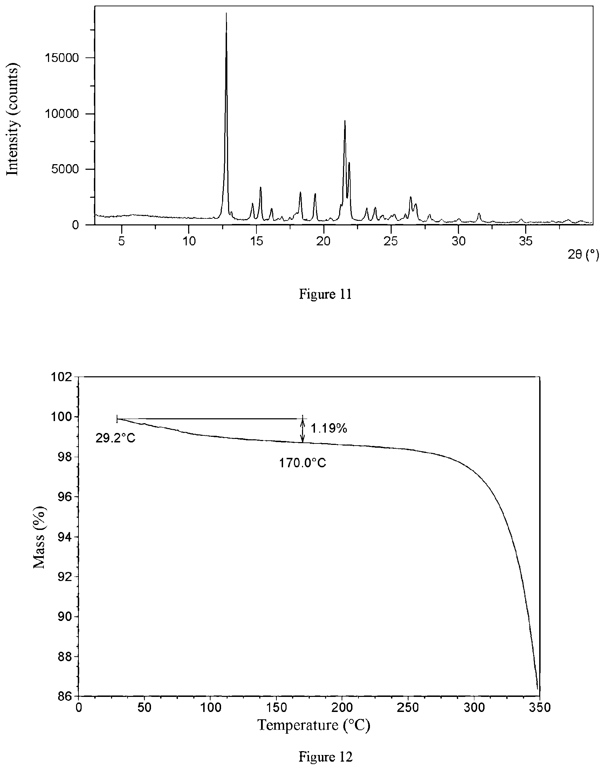

In an embodiment of the present invention, Crystalline Form E has an X-ray powder diffraction pattern as shown in Figure 11.

The process for preparing Crystalline Form E is characterized in that said process comprises:
step (1): heating the compound of formula (I) to 200°C-220°C,
step (2): then cooling to 0°C-5°C,
step (3): reheating to 140°C-160°C to obtain Crystalline Form E.

According to the process for preparing Crystalline Form E, in step (1), the heating is performed at a rate of 5°C/minute to 20°C/minute, for example, 10°C/minute.

According to the process for preparing Crystalline Form E, after the heating in step (1), the temperature is maintained for 5-20 minutes, for example, 10 minutes.

Through the step (1), the compound of formula (I) is melted.

According to the process for preparing Crystalline Form E, in step (2), the cooling is performed at a rate of 10°C/minute to 30°C/minute, for example, 20°C/minute. According to the process for preparing Crystalline Form E, after the cooling in step (2), the temperature is maintained for 1-5 minutes, for example, 2 minutes.

According to the process for preparing Crystalline Form E, after the cooling in step (2), an amorphous solid is obtained.

According to the process for preparing Crystalline Form E, in step (3), the heating is performed at a rate of 5°C/minute to 20°C/minute, for example, 10°C/minute.

Through the step (3), the recrystallization is performed.

### Processes for preparing Crystalline Form A

A process for preparing A-type crystal of a compound represented by formula (I) N-(((1S,3 S)-3-(methyl(7H-pyrrolo[2,3-d]pyrimidin-4-yl)amino)cyclobutyl)propane-1-sulf onamide, i.e., Crystalline Form A, is characterized in that, 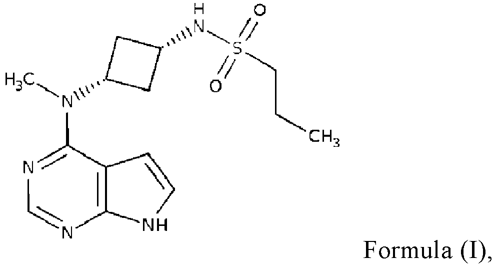 the compound of formula (I) is dissolved in a positive solvent, and after filtering, an anti-solvent is dropwisely added thereto to obtain Crystalline Form A.

In an embodiment of the present invention, the positive solvent is selected from at least one of dimethyl sulfoxide, dimethylformamide, dimethylacetamide, methylpyrrolidone, and trifluoroethanol, the anti-solvent is selected from at least one of pure water, methanol, ethanol, ethyl acetate, isopropyl acetate, dichloromethane, methyl tert-butyl ether, acetonitrile, acetone, and toluene.

In an embodiment of the present invention, the temperature for dissolving is 20°C to 30°C.

In an embodiment of the present invention, after filtering, the anti-solvent is dropwisely added with stirring.

In an embodiment of the present invention, the temperature for stirring is -25°C to 28°C, preferably -20°C to 20°C.

In an embodiment of the present invention, the stirring is performed for 1 day to 5 days until a solid precipitates, for example, for 2 days.

When a solid precipitates after the anti-solvent is added dropwise, the obtained solid is centrifuged, and the above-mentioned temperature for stirring is, for example, 22°C to 28°C. If no solid precipitates after an excessive amount of the anti-solvent is added, the dropwise addition of the anti-solvent is terminated, and the stirring is performed at -25°C to -10°C, preferably -20°C to -15°C, for example -20°C for 1 day to 5 days, for example 2 days.

A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, is characterized in that, 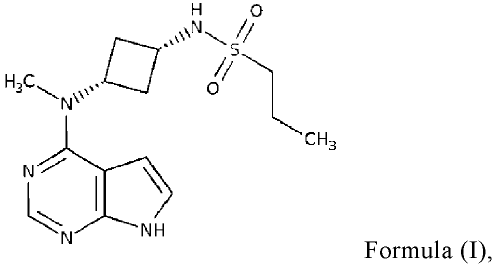 the compound of formula (I) is dissolved in a positive solvent, and after filtering, quickly added to an anti-solvent to obtain Crystalline Form A.

In an embodiment of the present invention, the positive solvent is selected from at least one of dimethylformamide and methylpyrrolidone, and the anti-solvent is selected from at least one of pure water and methyl tert-butyl ether.

In an embodiment of the present invention, the temperature for dissolving is 20°C to 30°C.

In an embodiment of the present invention, after filtering, the anti-solvent is quickly added with stirring.

In an embodiment of the present invention, the temperature for stirring is -20°C to 28°C. In an embodiment of the present invention, the stirring is performed until a solid precipitates.

If no solid precipitates after the positive solvent is added to the anti-solvent, the stirring is performed at -25°C to -10°C, preferably -20°C to -15°C, for example -20°C for 1 day to 5 days, for example 2 days.

A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, is characterized in that, 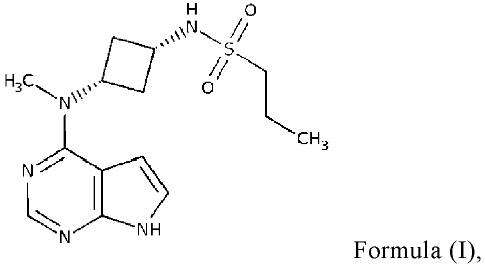 the compound of formula (I) is dissolved in at least one of methanol, tetrahydrofuran, 1,4-dioxane, trifluoroethanol, acetic acid, acetonitrile/pure water, methanol/dichloromethane, 40% aqueous dimethylamine solution and tetrahydrofuran/water, and after filtering, the solvent is volatilized until a solid precipitates to obtain Crystalline Form A.

In an embodiment of the present invention, the ratio by volume of acetonitrile/pure water is 1:1.

In an embodiment of the present invention, the ratio by volume of methanol/dichloromethane is 1:1.

In an embodiment of the present invention, the ratio by volume of tetrahydrofuran/water is 1:1.

In an embodiment of the present invention, a solid precipitates at 22°C to 28°C.

In an embodiment of the present invention, a high polymer is added after filtering.

The high polymer is selected from at least one of polyvinylpyrrolidone, polyvinyl alcohol, polyvinyl chloride, polyvinyl acetate, hydroxypropyl methylcellulose, methyl cellulose, polycaprolactone, polyethylene glycol, polymethyl methacrylate, sodium alginate and hydroxyethyl cellulose.

The high polymer is obtained by mixing polyvinylpyrrolidone, polyvinyl alcohol, polyvinyl chloride, polyvinyl acetate, hydroxypropyl methylcellulose and methyl cellulose at the weight ratio of 1:1:1:1:1:1.

The high polymer is obtained by mixing polycaprolactone, polyethylene glycol, polymethyl methacrylate, sodium alginate and hydroxyethyl cellulose at the weight ratio of 1:1:1:1:1.

The mass ratio of the high polymer to the compound of formula (I) is 1:4-10, or 1:6-8.

A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, is characterized in that, 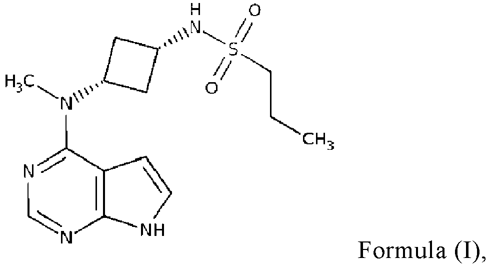 the compound of formula (I) is dissolved in an organic solvent or a mixed solvent system, filtered after reaching a dissolution equilibrium under a high temperature condition, and slowly cooled until a solid precipitates to obtain Crystalline Form A.

In an embodiment of the present invention, the organic solvent or the mixed solvent system is selected from acetonitrile, methanol/acetone, and acetonitrile/pure water.

In an embodiment of the present invention, the ratio by volume of methanol/acetone is 1:1.

In an embodiment of the present invention, the ratio by volume of acetonitrile/pure water is 1:1.

In an embodiment of the present invention, the high temperature is 40°C to 60°C, for example, 50°C.

In an embodiment of the present invention, the temperature for crystallizing is -20°C to 5°C.

In an embodiment of the present invention, the cooling rate is 0.05°C/minute to 0.5°C/minute.

After cooling to 2 to 10°C, for example 5°C, the system is allowed to stand for 1 day to 10 days, or 2 days to 5 days, for example 3 days while the temperature is kept at a constant temperature, and a solid precipitates. If no solid precipitates, the temperature is lowered to -25°C to -10°C, preferably -20°C to -15°C, for example -20°C, and the system is allowed to stand and a solid precipitates. The system is allowed to stand for 1 day to 10 days, or 3 days to 8 days, for example 7 days.

A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, is characterized in that, 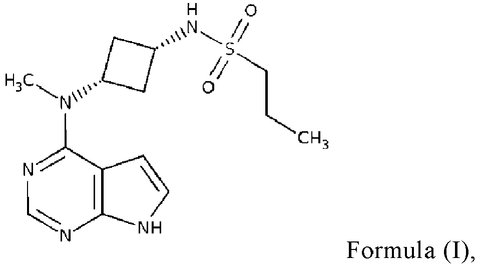 an excessive amount of the compound of formula (I) is added to an organic solvent or a mixed solvent system, and the suspension system is stirred to obtain Crystalline Form A. In an embodiment of the present invention, the organic solvent or the mixed solvent system is selected from at least one of methanol, isopropanol, ethyl acetate, ethyl lactate, acetonitrile, acetone, tetrahydrofuran, 2-methyltetrahydrofuran, ethanol/pure water, 2-butanol/pure water, isopropyl acetate/dimethyl sulfoxide, methyl isobutyl ketone/benzyl alcohol, methyl tert-butyl ether/acetic acid, chlorobenzene/dimethylformamide, cyclohexanol/methylpyrrolidone, n-octanol/trifluoroethanol, ethylene glycol dimethylether/trifluoroethanol, 40% aqueous dimethylamine solution, and acetonitrile/pure water.

In an embodiment of the present invention, the ratio by volume of ethanol/pure water is 1:1, the ratio by volume of 2-butanol/pure water is 1:1, the ratio by volume of isopropyl acetate/dimethyl sulfoxide is 5:1, the ratio by volume of methyl isobutyl ketone/benzyl alcohol is 1:1, the ratio by volume of methyl tert-butyl ether/acetic acid is 3:1, the ratio by volume of chlorobenzene/dimethylformamide is 3:1, the ratio by volume of cyclohexanol/methylpyrrolidone is 2:1, the ratio by volume of n-octanol/trifluoroethanol is 2:1, the ratio by volume of ethylene glycol dimethylether/trifluoroethanol is 2:1, water activity of acetonitrile/pure water is 0.2-1.0.

In an embodiment of the present invention, the temperature is 25°C to 50°C.

A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, is characterized in that, 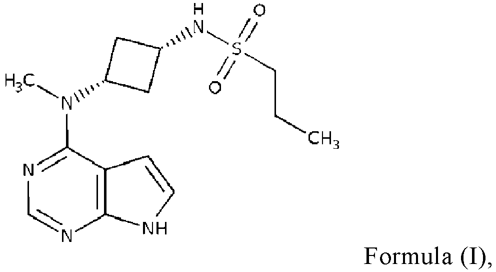 the compound of formula (I) is dissolved in a positive solvent, and placed under an anti-solvent atmosphere to perform the liquid vapor diffusion, and then the resulting system is volatilized and separated to obtain Crystalline Form A.

In an embodiment of the present invention, the positive solvent is selected from at least one of acetic acid/methanol, dimethylformamide/acetone, dimethyl sulfoxide/acetone, dimethyl sulfoxide/acetonitrile.

In an embodiment of the present invention, the ratio by volume of acetic acid/methanol is 1:3, the ratio by volume of dimethylformamide/acetone is 1:4, the ratio by volume of dimethyl sulfoxide/acetone is 1:4, the ratio by volume of dimethyl sulfoxide/acetonitrile is 1:4.

In an embodiment of the present invention, the temperature is 22°C to 28°C.

In an embodiment of the present invention, the liquid vapor diffusion is performed for 2 days to 20 days, or 4 days to 16 days, for example 12 days.

In an embodiment of the present invention, the anti-solvent is selected from at least one of methanol, acetone, and acetonitrile.

### Crystalline Form F

F-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form F, is characterized in that, by using the Cu-Kα radiation, Crystalline Form F has an X-ray powder diffraction pattern comprising characteristic peaks at the 2θ values of 10.9°±0.2°, 14.8°±0.2°, and 20.6°±0.2°,

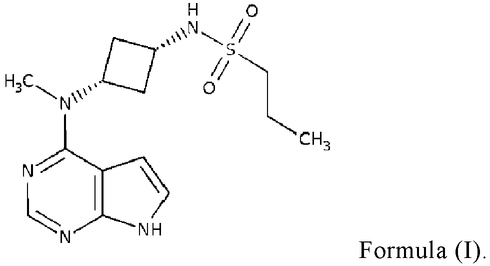

In an embodiment of the present invention, Crystalline Form F has an X-ray powder diffraction pattern comprising characteristic peak(s) at one or two 2θ values of 22.0°±0.2°, 24.9°±0.2°.

In an embodiment of the present invention, Crystalline Form F has an X-ray powder diffraction pattern comprising characteristic peaks at the 2θ values of 22.0°±0.2°, and 24.9°±0.2°.

In an embodiment of the present invention, Crystalline Form F has an X-ray powder diffraction pattern comprising characteristic peak(s) at one, two, or three 2θ values of 18.9°±0.2°, 21.6°±0.2°, 25.8°±0.2°.

In an embodiment of the present invention, Crystalline Form F has an X-ray powder diffraction pattern comprising characteristic peaks at the 2θ values of 18.9°±0.2°, 21.6°±0.2°, and 25.8°±0.2°.

In an embodiment of the present invention, Crystalline Form F has an X-ray powder diffraction pattern comprising characteristic peaks at any four, or five, or six, or seven, or eight 2θ values of 10.9°±0.2°, 14.8°±0.2°, 18.9°±0.2°, 20.6°±0.2°, 21.6°±0.2°, 22.0°±0.2°, 24.9°±0.2°, and 25.8°±0.2°.

In an embodiment of the present invention, Crystalline Form F has an X-ray powder diffraction pattern comprising characteristic peaks at the 2θ values of 10.9°±0.2°, 14.8°±0.2°, 18.9°±0.2°, 22.0°±0.2°, and 25.8°±0.2°.

In an embodiment of the present invention, Crystalline Form F has an X-ray powder diffraction pattern comprising characteristic peaks at the 2θ values of 10.9°±0.2°, 14.8°±0.2°, 18.9°±0.2°, 20.6°±0.2°, 22.0°±0.2°, 24.9°±0.2°, and 25.8°±0.2°.

In an embodiment of the present invention, Crystalline Form F has an X-ray powder diffraction pattern comprising characteristic peaks at the 2θ values of 10.9°±0.2°, 14.8°±0.2°, 18.9°±0.2°, 20.6°±0.2°, 21.6°±0.2°, 22.0°±0.2°, 24.9°±0.2°, and 25.8°±0.2°. In an embodiment of the present invention, Crystalline Form F has an X-ray powder diffraction pattern comprising characteristic peaks at the 2θ values of 9.1°±0.2°, 10.9°±0.2°, 14.8°±0.2°, 18.9°±0.2°, 20.6°±0.2°, 21.6°±0.2°, 22.0°±0.2°, 24.9°±0.2°, and 25.8°±0.2°.

In an embodiment of the present invention, Crystalline Form F has an X-ray powder diffraction pattern comprising characteristic peaks at any four, or five, or six, or seven, or eight, or nine 2θ values of 10.9°±0.2°, 11.3°±0.2°, 14.8°±0.2°, 18.9°±0.2°, 20.6°±0.2°, 21.6°±0.2°, 22.0°±0.2°, 24.9°±0.2°, and 25.8°±0.2°.

In an embodiment of the present invention, Crystalline Form F has an X-ray powder diffraction pattern comprising characteristic peaks at the 2θ values of 10.9°±0.2°, 11.3°±0.2°, 14.8°±0.2°, 18.9°±0.2°, 22.0°±0.2°, and 25.8°±0.2°.

In an embodiment of the present invention, Crystalline Form F has an X-ray powder diffraction pattern comprising characteristic peaks at the 2θ values of 10.9°±0.2°, 11.3°±0.2°, 14.8°±0.2°, 18.9°±0.2°, 20.6°±0.2°, 22.0°±0.2°, 24.9°±0.2°, and 25.8°±0.2°.

In an embodiment of the present invention, Crystalline Form F has an X-ray powder diffraction pattern comprising characteristic peaks at the 2θ values of 10.9°±0.2°, 11.3°±0.2°, 14.8°±0.2°, 18.9°±0.2°, 20.6°±0.2°, 21.6°±0.2°, 22.0°±0.2°, 24.9°±0.2°, and 25.8°±0.2°.

In an embodiment of the present invention, Crystalline Form F has an X-ray powder diffraction pattern comprising characteristic peaks at any four, or five, or six, or seven, or eight, or nine 2θ values of 10.9°±0.2°, 14.8°±0.2°, 18.3°±0.2°, 18.9°±0.2°, 20.6°±0.2°, 21.6°±0.2°, 22.0°±0.2°, 24.9°±0.2°, and 25.8°±0.2°.

In an embodiment of the present invention, Crystalline Form F has an X-ray powder diffraction pattern comprising characteristic peaks at the 2θ values of 10.9°±0.2°, 14.8°±0.2°, 18.3°±0.2°, 18.9°±0.2°, 20.6°±0.2°, 21.6°±0.2°, 22.0°±0.2°, 24.9°±0.2°, and 25.8°±0.2°.

In an embodiment of the present invention, Crystalline Form F has an X-ray powder diffraction pattern comprising characteristic peaks at any four, or five, or six, or seven, or eight, or nine, or ten 2θ values of 10.9°±0.2°, 11.3°±0.2°, 14.8°±0.2°, 18.3°±0.2°, 18.9°±0.2°, 20.6°±0.2°, 21.6°±0.2°, 22.0°±0.2°, 24.9°±0.2°, and 25.8°±0.2°.

In an embodiment of the present invention, Crystalline Form F has an X-ray powder diffraction pattern comprising characteristic peaks at the 2θ values of 10.9°±0.2°, 11.3°±0.2°, 14.8°±0.2°, 18.3°±0.2°, 18.9°±0.2°, 20.6°±0.2°, 21.6°±0.2°, 22.0°±0.2°, 24.9°±0.2°, and 25.8°±0.2°.

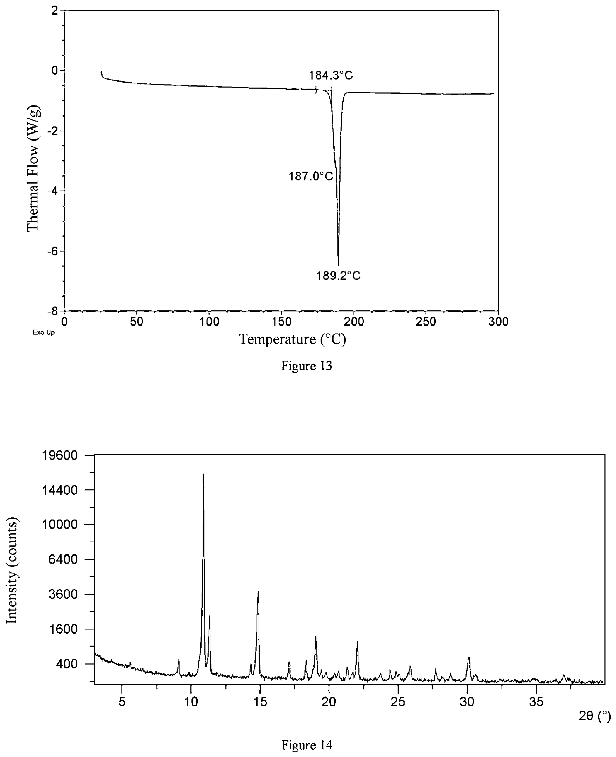

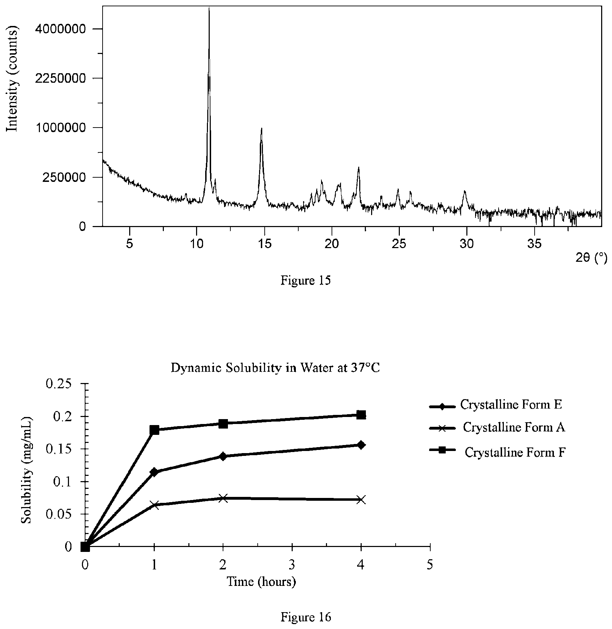

In an embodiment of the present invention, Crystalline Form F has an X-ray powder diffraction pattern as shown in Figure 14 or Figure 15.

A process for preparing Crystalline Form F is characterized in that, a solid of the compound of formula (I) is heated, and then cooled to obtain Crystalline Form F.

In an embodiment of the present invention, the above-mentioned solid is heated at a rate of 5-20°C/minute, for example 10°C/minute to 200-220°C, for example 210°C.

In an embodiment of the present invention, after heating, the temperature is maintained for 5 to 20 minutes, for example, 10 minutes.

In an embodiment of the present invention, the temperature is lowered to 0-5°C, for example 0°C, at a rate of 5-20°C/minute, for example 10°C/minute.

A process for preparing Crystalline Form F is characterized in that, the compound of formula (I) is added to methanol/dichloromethane, and after filtering, the filtrate is subjected to the liquid vapor diffusion with methyl tert-butyl ether to obtain Crystalline Form F.

According to the process for preparing Crystalline Form F, the ratio by volume of methanol/dichloromethane is 0.5-2:0.5-2, for example the ratio by volume of methanol/dichloromethane is 1:1.

The process for preparing Crystalline Form F is performed at a temperature of 22°C to 28°C, preferably at room temperature.

According to the process for preparing Crystalline Form F, the liquid vapor diffusion is performed for 1 day to 20 days, or 2 days to 10 days, for example 3 days.

According to the present invention, the compound of formula (I) as raw material refers to its solid (crystal or amorphous), semi-solid, wax or oil form. Preferably, the compound of formula (I) as raw material is in the form of solid powder. The "stirring" is completed by conventional methods in the art, such as magnetic stirring or mechanical stirring, and the stirring speed is 50-1800 rpm, wherein the stirring speed for the magnetic stirring is 200-1500 rpm, preferably 300-1000 rpm, and the stirring speed for the mechanical stirring is preferably 100-300 rpm.

The above-mentioned crystals of the present invention (Crystalline Forms B, C, D, E, and F of the compound of formula (I)) can be used to prepare pharmaceutical compositions, and the above-mentioned crystals of the present invention and pharmaceutically acceptable carriers are contained during the preparation of pharmaceutical compositions. The above-mentioned crystals of the present invention (Crystalline Forms B, C, D, E, and F of the compound of formula (I)) can be used to prepare pharmaceutical compositions having JAK1 inhibitory activity, which contain the above-mentioned crystals of the present invention (Crystalline Forms B, C, D, E, and F of the compound of formula (I)) as an active component. The above-mentioned crystals of the present invention (Crystalline Forms B, C, D, E, and F of the compound of formula (I)) can be used to manufacture a preventing or treating drug for atopic dermatitis, lupus vulgavis and/or plaque psoriasis, which contains the above-mentioned crystals of the present invention (Crystalline Forms B, C, D, E, and F of the compound of formula (I)) as an active component.

The present invention also provides a pharmaceutical composition containing any of the above-mentioned crystals of the present invention (Crystalline Forms B, C, D, E, and F of the compound of formula (I)) and a pharmaceutically acceptable carrier.

The present invention also provides a pharmaceutical composition having JAK1 inhibitory activity, which contains any of the above-mentioned crystals of the present invention (Crystalline Forms B, C, D, E, and F of the compound of formula (I)) as an active component.

The present invention provides a preventing or treating drug for atopic dermatitis, lupus vulgavis and/or plaque psoriasis, which contains any of the above-mentioned crystals of the present invention (Crystalline Forms B, C, D, E, and F of the compound of formula (I)) as an active component.

In the present invention, "crystal" or "crystalline form" refers to the crystal or the crystalline form being identified by the X-ray diffraction pattern shown herein. Those skilled in the art are able to understand that physicochemical properties discussed herein can be characterized. The experimental errors depend on the instrument conditions, the sampling processes and the purity of samples. In particular, those skilled in the art generally know that the X-ray diffraction pattern typically varies with the experimental conditions. In particular, it is necessary to point out that, the relative intensity of the diffraction peaks in the X-ray diffraction pattern may also vary with the experimental conditions; therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and absolute comparison is not required. In addition, the experimental error of the diffraction peak position is usually 5% or less, and the error of these positions should also be taken into account. An error of ±0.2°is usually allowed. In addition, due to experimental factors such as sample thickness, the overall offset of the diffraction peak is caused, and a certain offset is usually allowed. Thus, it will be understood by those skilled in the art that a crystalline form of the present invention is not necessarily to have the exactly same X-ray diffraction pattern of the example shown herein and the "X-ray powder diffraction pattern is same" as described herein is not meaning absolutely the same, the same peak position can differ by ±0.2°and the peak intensity allows for some variability. Any crystalline forms whose X-ray diffraction patterns have the same or similar characteristic peaks should be within the scope of the present invention. Those skilled in the art can compare the patterns shown in the present invention with that of an unknown crystalline form in order to identify whether these two groups of patterns reflect the same or different crystalline forms.

In some embodiments, Crystalline Forms B, C, D, E, and F of the present invention are pure, single, and substantially free of any other crystalline forms. In the present invention, the term "substantially free" when used to describe a novel crystalline form, it means that the content of other crystalline forms in the novel crystalline form is less than 20% (w/w), specifically less than 10% (w/w), more specifically less than 5% (w/w) and further more specifically less than 1% (w/w). It should be noted that a numerical value and a numerical range in the present invention should not be narrowly understood as the numerical value itself or the numerical range itself. It should be understood by those skilled in the art that the specific numerical value can be floated according to the specific technical environment on the basis of not departing from the spirit and principle of the present invention. In the present invention, the number of floating ranges which can be expected by one of skilled in the art is represented by the term "about".

The upper limit value and the lower limit value of the numerical range described in the specification of the present invention can be arbitrarily combined.

### Examples

The following will further illustrate the present invention through specific examples, which are not intended to limit the protection scope of the present invention. Those skilled in the art can make improvements to the preparation processes and the used instruments within the scope of the claims, and those improvements should be considered as falling into the protection scope of the present invention. Therefore, the protective scope of the present invention should be defined by the appended claims.

In the present invention, "room temperature" generally refers to 22°C to 28°C, unless otherwise specified.

The abbreviations used in the present invention are explained as follows:
XRPD : X-ray Powder Diffraction
DSC : Differential Scanning Calorimetry analysis
TGA : ThermoGravimetric Analysis
¹H NMR: Nuclear Magnetic Resonance Hydrogen spectrum
DVS: Dynamic Vapor Sorption
PSD: Particle Size Distribution
PLM : Polarized Light Microscope
HPLC: High Performance Liquid Chromatography.

X-ray powder diffraction patterns of the present invention were collected on Empyrean-Type and X' Pert³-Type X-ray powder diffractometers of Panalytical Corporation. The process parameters of X-ray powder diffraction of the present invention were as follows:
X-ray source: Cu, Kα
Kα1 (Å): 1.54060; Kα2 (Å): 1.54443
Kα2/Kα1intensity ratio: 0.50
Voltage: 45 kilovolts (kV)
Current: 40 milliamps (mA)
Scanning range: from 3.0 to 40.0 degrees (2θ angle).

Differential scanning calorimetry analysis charts of the present invention were collected on the Q200-type and Discovery DSC 2500-type differential scanning calorimeters of TA Company. The process parameters of the differential scanning calorimetry analysis of the present invention were as follows:
Scanning rate: 10°C/minute
Protection gas: Nitrogen gas.

The thermogravimetric analysis charts of the present invention were collected on Discovery TGA 5500-type and Q5000-type thermogravimetric analyzers of TA Company. The process parameters of the thermogravimetric analysis of the present invention were as follows:
Scanning rate: 10°C/minute
Protection gas: Nitrogen gas.

Nuclear magnetic resonance hydrogen spectrum data (¹H NMR) of the present invention were collected from a Bruker Avance II DMX 400M HZ nuclear magnetic resonance spectrometer. 1-5 mg of a sample was weighed, and dissolved in 0.5 mL of deuterated dimethyl sulfoxide to formulate 2-10 mg/mL of a solution for test.

The dynamic vapor sorption diagrams of the present invention were collected on Intrinsic-type and Intrinsic Plus-type dynamic vapor sorption instruments of SMS company. The process parameters of the dynamic vapor sorption test of the present invention were as follows:
Temperature: 25°C
Protection gas and its flow rate: N₂, 200 mL/minute
dm/dt: 0.002%/minute
Minimum dm/dt equilibration time: 10 minutes
Maximum Equilibration Time: 180 minutes
Relative humidity range: 0%RH-95%RH-0%RH
Relative humidity gradient: 10% (0%RH-90%RH-0%RH), 5% (90%RH-95%RH and 95%RH-90%RH).

The particle size distribution results of the present invention were collected on S3500-type laser particle size analyzer of the Microtrac company. The Microtrac S3500 was equipped with an SDC (Sample Delivery Controller) sampling system. The wet method was used in this test, using Isopar G (containing 0.2% lecithin) as the test dispersion medium. The process parameters of the laser particle size analyzer were as follows.

| | |
|---|---|
| Particle size distribution: volume distribution | Acquisition time: 10 seconds |
| Dispersion medium: Isopar G | Particle size coordinate: Standard |
| Collection times: 3 times | Refractive index of dispersion medium: 1.42 |
| Transparency: Transparent | Residuals: Enabled |
| Particle refractive index: 1.59 | Flow rate: 60%* |
| Particle shape: Irregular | Filtration: Enabled |
| Ultrasonication power: 30 W | Ultrasonication time: 30 seconds |

| | |
|---|---|
| ^{∗}: Flow rate 60% was 60% of 65 mL/s. | |

The intrinsic dissolution rate data of the present invention were collected on an Agilent 708DS-type dissolution apparatus of the Agilent company. The intrinsic dissolution test conditions were as follows.

| | |
|---|---|
| Dissolution apparatus | Agilent 708DS |
| Method | Paddle |
| Medium | pH 6.8 Phosphate Buffer |
| Medium volume | 900 mL |
| Rotation speed | 100 rpm |
| Medium temperature | 37°C |
| Sampling points | 1, 2, 3, 4, 5, 10, 15, 20, 25, 30 minutes |
| Supplemental medium | No |

The polarized light microscope photos of the present invention were taken at room temperature with a Zeiss microscope Axio Scope.A1 equipped with an Axiocam 305 color camera and 5×, 10×, 20× and 50× objective lenses.

### Preparation Example 1: Compound Synthesis

The synthesis route of the compound formula (I) was performed in five steps.

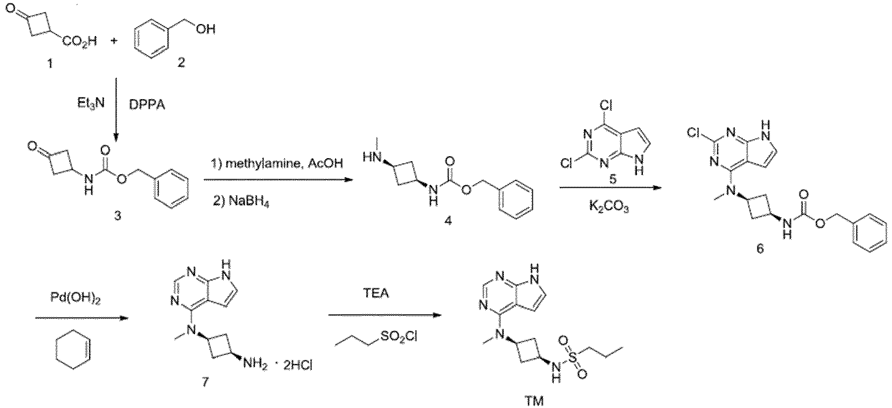

Step 1: Under the nitrogen gas protection, 30 g (0.26 mol, 1.0 eq.) of the dried Compound 1 and 28.4 g (0.26 mol, 1.0 eq.) of Compound 2 were weighed and dissolved in 240 mL of a mixed solvent of dried tetrahydrofuran/toluene (1:1, v/v), and then 34.2 g (0.34 mol, 1.3 eq.) of triethylamine and 4A molecular sieve were added thereto. The temperature was raised to an external temperature of 90°C, and 71.5 g (0.26 mol, 1.0 eq.) of diphenylphosphoryl azide was added dropwise to the system under reflux (10 minutes). After the dropwise addition, the reaction solution was brown-black. The temperature was slowly lowered to 75°C, and the reaction was performed at this temperature for 7 hours. After the reaction was completed, the reaction solution was concentrated under reduced pressure to obtain a black viscous substance. The obtained black viscous substance was re-dissolved in ethyl acetate, washed with saturated sodium carbonate solution and 1M hydrochloric acid solution successively to remove impurities, and then anhydrous sodium sulfate was added to remove water. The resulting mixture was filtered and rotary-evaporated under reduced pressure to give a brown-black viscous substance. Finally, the obtained brown-black viscous substance was separated by silica gel column chromatography (petroleum ether/ethyl acetate=6/1-3/1) to produce 50.1 g of a light brown solid (Compound 3).

Step 2: 39.0 g (0.18 mol, 1.0 eq.) of Compound 3 was weighed and dissolved in 320 mL of ethanol, and 20 mL of acetic acid and 256.7 g (11.6 eq.) of an aqueous methylamine(monomethylamine) solution were added thereto. The reaction system was sealed and stirred at room temperature for 5 hours. Then, 15.5 g (2.3 eq.) of sodium borohydride was added in portions under an ice bath condition, and the mixture was stirred at room temperature overnight. After the reaction was completed, 40 mL of saturated ammonium chloride solution was added to quench the reaction. Subsequently, the reaction solution was rotary-evaporated to remove ethanol. Under an ice bath condition, the pH of the system was adjusted to about 3 with concentrated hydrochloric acid, and washed with ethyl acetate to remove impurities. Then the pH of the system was adjusted with 10% sodium hydroxide solution to about 10, the product was extracted with ethyl acetate, anhydrous sodium sulfate was added to remove water, and the resulting mixture was filtered and rotary-evaporated under reduced pressure to give a crude product. The crude product was dissolved in 50 mL of dichloromethane, 20 mL of 4M hydrogen chloride/dioxane solution was added under an ice bath condition, and the precipitated solid was separated. 100 mL of isopropanol was added to the precipitated solid, the mixture was stirred for 3 hours, filtered under reduced pressure and dried to obtain 19.5 g of a white solid (Compound 4).

Step 3: 22.5 g (0.16 mol, 2.6 eq.) of potassium carbonate was weighed and dissolved in 150 mL of pure water, the resulting mixture was stirred at room temperature and 14.7 g (0.06 mol, 1.0 eq.) of Compound 4 and 11.5 g (0.06 mol, 0.98 eq.) of Compound 5 were successively added thereto. The temperature was raised to 95°C and the reaction mixture was stirred for 10 hours. After the reaction was completed, the mixture was cooled, filtered, and washed with pure water to obtain a pale yellow solid. 40 mL of methanol was added to the obtained solid, the resulting mixture was stirred for 2 hours, filtered under reduced pressure and dried to obtain 19.4 g of a white solid (Compound 6).

Step 4: 26.5 g (0.07 mol, 1.0 eq.) of Compound 6 was weighed and suspended in 500 mL of ethanol, and then 13.5 g (0.10 mol, 1.21 eq.) of palladium hydroxide and 181.4 g (2.21 mol, 22 eq.) of cyclohexene were added. The temperature was raised to 82°C and the reaction mixture was stirred for 5 hours. After the reaction was completed, the reaction mixture was cooled, filtered, washed with ethanol, and concentrated under reduced pressure until a solid precipitated. 100 mL of acetonitrile was added to the obtained solid, the resulting mixture was stirred for 1 hour, filtered under reduced pressure and dried to obtain 9.5 g of a pale yellow solid (Compound 7).

Step 5: 10.0 g (0.04 mol, 1.0 eq.) of Compound 7 was weighed and suspended in 200 mL of tetrahydrofuran, then 23.9 g (0.24 mol, 6.0 eq.) of triethylamine was added, and the resulting mixture was stirred at room temperature for 1 hour. Subsequently, 6.76 g (0.05 mol, 1.2 eq.) of propylsulfonyl chloride was slowly added dropwise under an ice bath condition, and the resulting mixture was stirred at room temperature overnight. After the reaction was completed, pure water was added to the reaction solution, the reaction product was extracted with dichloromethane, and a yellow crude product was obtained by rotary-evaporation under reduced pressure. Finally, the obtained crude product was separated by silica gel column chromatography (dichloromethane/methanol=50/1-30/1) to produce 3.7 g of a light yellow solid (the compound of formula (I)).

### Preparation Example 2: Preparation of Crystalline Form A

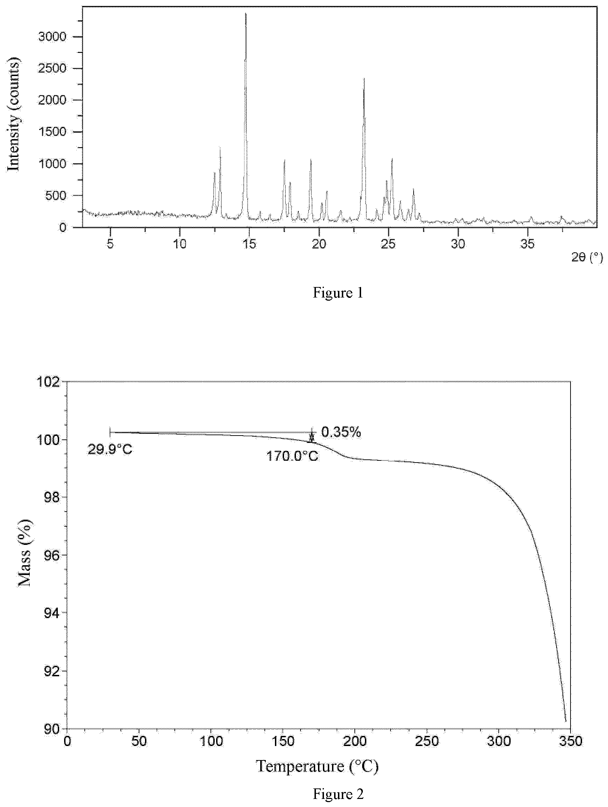

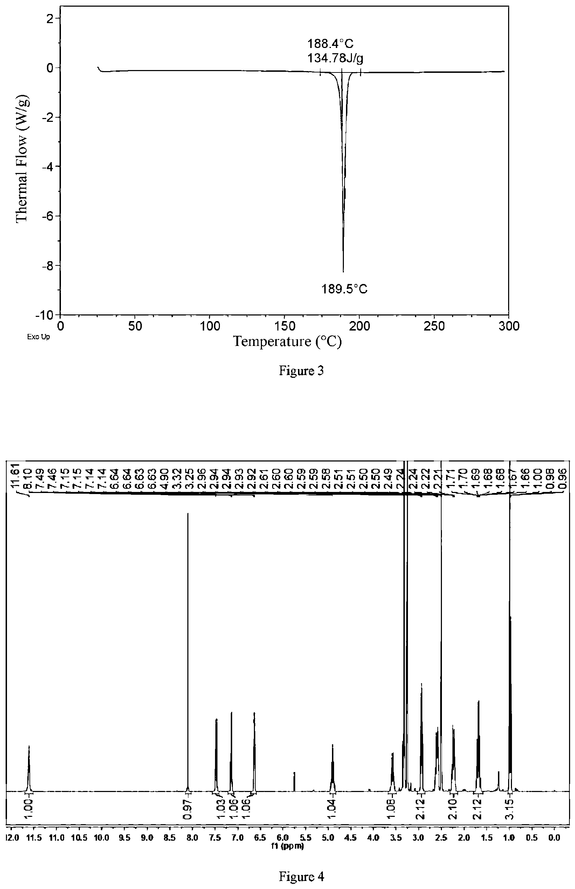

By detection, the solid obtained in Preparation Example 1 was Crystalline Form A, its X-ray powder diffraction data was shown in Table 1, the diffraction pattern was shown in Figure 1, and TGA, DSC and ¹H NMR data were shown in Figures 2 to 4, respectively.

**Table 1**

| **Diffraction angle 2θ** | **d value** | **Intensity %** |
|---|---|---|
| 8.70 | 10.16 | 1.72 |
| 12.48 | 7.09 | 21.67 |
| 12.89 | 6.87 | 33.39 |
| 13.31 | 6.65 | 1.84 |
| 14.73 | 6.01 | 100.00 |
| 15.76 | 5.62 | 3.58 |
| 16.48 | 5.38 | 2.55 |
| 17.52 | 5.06 | 28.05 |
| 17.93 | 4.95 | 18.32 |
| 18.53 | 4.79 | 4.62 |
| 19.39 | 4.58 | 28.79 |
| 20.19 | 4.40 | 8.39 |
| 20.55 | 4.32 | 14.28 |
| 21.55 | 4.12 | 5.34 |
| 22.21 | 4.00 | 1.66 |
| 23.23 | 3.83 | 67.83 |
| 24.17 | 3.68 | 5.62 |
| 24.70 | 3.60 | 11.39 |
| 24.89 | 3.58 | 19.33 |
| 25.26 | 3.53 | 30.06 |
| 25.85 | 3.45 | 9.58 |
| 26.43 | 3.37 | 6.05 |
| 26.78 | 3.33 | 15.97 |
| 27.19 | 3.28 | 4.10 |
| 29.80 | 3.00 | 1.96 |
| 30.27 | 2.95 | 1.80 |
| 31.84 | 2.81 | 2.28 |
| 32.66 | 2.74 | 0.78 |
| 33.99 | 2.64 | 1.05 |
| 35.24 | 2.55 | 2.99 |
| 37.42 | 2.40 | 3.51 |
| 38.25 | 2.35 | 1.17 |
| 39.43 | 2.29 | 1.50 |

### Examples 1-4: Preparation of Crystalline Form A (Liquid Vapor Diffusion-Room Temperature Vaporation Method)

An appropriate amount of a solid of the compound of formula (I) obtained in Preparation Example 1 was weighed and placed into a 3 mL glass vial at room temperature, a corresponding volume of a positive solvent was added to dissolve the solid, the sample solution was filtered with a polytetrafluoroethylene filter membrane having a pore size of 0.45 microns into a new 3 mL glass vial, which was placed open into a 20 mL glass bottle prefilled with 4 mL of a corresponding anti-solvent. The bottle was sealed, and then placed at room temperature for 12 days to perform the liquid vapor diffusion. After that, the sample was transferred to room temperature and placed open for the volatilization until a solid precipitated.

By detection, all of the solids obtained in these examples were Crystalline Form A. The detailed test conditions involved in these examples were shown in Table 2, and the X-ray powder diffraction data of the sample of Example 1 was shown in Table 3.

**Table 2**

| **Example** | **Solid Mass (mg)** | **Positive solvent** | | **Anti-solvent** | **Volatilization time (days)** |
|---|---|---|---|---|---|
| | | **Type (v/v)** | **Volume (mL)** | | |
| 1 | 15.2 | Acetic acid/ Methanol (1:3) | 0.8 | Methanol | 22 |
| 2 | 15.3 | Dimethylformamide/ Acetone(1:4) | 1.0 | Acetone | 7 |
| 3 | 14.8 | Dimethyl sulfoxide/ Acetone(1:4) | 1.0 | Acetone | 17 |
| 4 | 15.4 | Dimethyl sulfoxide/ Acetonitrile(1:4) | 0.7 | Acetonitrile | 17 |

**Table 3**

| **Diffraction angle 2θ** | **d value** | **Intensity %** |
|---|---|---|
| 8.69 | 10.18 | 0.93 |
| 12.48 | 7.09 | 7.86 |
| 12.87 | 6.88 | 17.68 |
| 13.33 | 6.64 | 1.10 |
| 14.73 | 6.01 | 100.00 |
| 15.75 | 5.63 | 1.98 |
| 16.48 | 5.38 | 2.37 |
| 17.52 | 5.06 | 12.44 |
| 17.94 | 4.95 | 12.35 |
| 18.52 | 4.79 | 1.95 |
| 19.39 | 4.58 | 16.99 |
| 20.20 | 4.40 | 2.02 |
| 20.54 | 4.32 | 19.33 |
| 21.54 | 4.13 | 5.51 |
| 23.23 | 3.83 | 45.91 |
| 24.17 | 3.68 | 7.20 |
| 24.67 | 3.61 | 2.94 |
| 24.88 | 3.58 | 15.11 |
| 25.26 | 3.53 | 11.97 |
| 25.86 | 3.45 | 8.45 |
| 26.41 | 3.37 | 2.65 |
| 26.79 | 3.33 | 3.41 |
| 27.19 | 3.28 | 2.61 |
| 29.84 | 2.99 | 1.13 |
| 30.23 | 2.96 | 0.51 |
| 31.55 | 2.84 | 1.74 |
| 31.84 | 2.81 | 2.1 |
| 32.99 | 2.72 | 1.00 |
| 34.04 | 2.63 | 0.74 |
| 34.84 | 2.58 | 1.32 |
| 35.23 | 2.55 | 3.14 |
| 36.45 | 2.46 | 0.94 |
| 37.43 | 2.40 | 2.23 |
| 38.22 | 2.35 | 0.81 |
| 39.47 | 2.28 | 0.81 |

### Examples 5-9: Preparation of Crystalline Form A (Slow Volatilization Method)

An appropriate amount of a solid of the compound of formula (I) obtained in Preparation Example 1 was weighed and placed into a 3 mL glass vial at room temperature, and a corresponding volume of a solvent was added to dissolve the solid. The sample solution was filtered with a polytetrafluoroethylene filter membrane having a pore size of 0.45 microns into a new 3 mL glass vial, and about 2 mg of a mixed high polymer was added to a selected part of the system to induce the crystallization of the compound of formula (I). The vial was sealed with a sealing membrane, 4 pinholes were pricked on the membrane, and then the vial was placed at room temperature for slow volatilization until a solid precipitated.

By detection, all of the solids obtained in these examples were Crystalline Form A. The detailed test conditions involved in these examples were shown in Table 4, and the X-ray powder diffraction data of the sample of Example 6 was shown in Table 5.

**Table 4**

| **Example** | **Solid Mass (mg)** | **Solvent** | | **Kind of high polymer** |
|---|---|---|---|---|
| | | **Type (v/v)** | **Volume (mL)** | |
| 5 | 15.4 | Acetonitrile/ Water (1:1) | 2.4 | Not added |
| 6 | 14.7 | Methanol/Dichloro methane (1:1) | 1.6 | Not added |
| 7 | 15.5 | Acetonitrile/ Water (1:1) | 2.4 | Mixed high polymer A |
| 8 | 15.3 | Tetrahydrofuran/ Water (1:1) | 2.4 | Mixed high polymer B |
| 9 | 14.9 | Methanol/Dichloro methane (1:1) | 1.6 | Mixed high polymer B |

| | | | | |
|---|---|---|---|---|
| Mixed high polymer A: polyvinylpyrrolidone, polyvinyl alcohol, polyvinyl chloride, polyvinyl acetate, hydroxypropyl methylcellulose and methyl cellulose (mixed at 1:1:1:1:1:1 by mass) Mixed high polymer B: polycaprolactone, polyethylene glycol, polymethyl methacrylate, sodium alginate and hydroxyethyl cellulose (mixed at 1:1:1:1:1 by mass) | | | | |

**Table 5**

| **Diffraction angle 2θ** | **d value** | **Intensity %** |
|---|---|---|
| 8.68 | 10.19 | 0.69 |
| 12.48 | 7.09 | 3.71 |
| 12.89 | 6.87 | 2.38 |
| 14.73 | 6.01 | 100.00 |
| 15.76 | 5.62 | 2.85 |
| 16.48 | 5.38 | 2.79 |
| 17.52 | 5.06 | 10.74 |
| 17.94 | 4.94 | 17.03 |
| 18.53 | 4.79 | 3.03 |
| 19.40 | 4.58 | 23.60 |
| 20.20 | 4.40 | 1.36 |
| 20.55 | 4.32 | 1.45 |
| 21.56 | 4.12 | 1.70 |
| 22.96 | 3.87 | 5.45 |
| 23.24 | 3.83 | 52.64 |
| 24.18 | 3.68 | 4.21 |
| 24.89 | 3.58 | 2.52 |
| 25.27 | 3.52 | 14.69 |
| 25.85 | 3.45 | 5.68 |
| 26.44 | 3.37 | 2.73 |
| 26.78 | 3.33 | 4.50 |
| 27.20 | 3.28 | 4.50 |
| 28.59 | 3.12 | 0.27 |
| 31.36 | 2.85 | 1.39 |
| 32.61 | 2.75 | 0.30 |
| 34.07 | 2.63 | 0.74 |
| 35.17 | 2.55 | 0.30 |
| 36.48 | 2.46 | 0.36 |
| 37.42 | 2.40 | 2.22 |
| 39.51 | 2.28 | 0.60 |

### Examples 10-12: Preparation of Crystalline Form A (Slow Cooling Method)

An appropriate amount of a solid of the compound of formula (I) obtained in Preparation Example 1 was weighed and placed into a 3 mL glass vial at room temperature, and a corresponding volume of a solvent was added to produce a suspension. The suspension was magnetically stirred at 50°C (about 1000 rpm) for about two hours, then, while hot, the sample solution was filtered with a polytetrafluoroethylene filter membrane having a pore size of 0.45 microns into a new 3 mL glass vial. The vial was sealed, cooled down from 50°C to 5°C at a rate of 0.1°C per minute, and then allowed to stand at a constant temperature of 5°C for about three days. If no solid precipitated, the sample was transferred to -20°C and allowed to stand for about seven days to obtain a precipitated solid.

By detection, all of the solids obtained in these examples were Crystalline Form A. The detailed test conditions involved in these examples were shown in Table 6, and the X-ray powder diffraction data of the sample of Example 10 was shown in Table 7.

**Table 6**

| **Example** | **Solid Mass (mg)** | **Solvent (v/v)** | **Volume (mL)** | **Conditions** |
|---|---|---|---|---|
| 10 | 15.7 | Acetonitrile/ water (1:1) | 0.8 | Stand at 5°C for crystallization |
| 11 | 15.7 | Methanol/ acetone(1: 1) | 0.8 | Stand at -20°C for crystallization |
| 12 | 15.0 | Acetonitrile | 2.0 | Stand at -20°C for crystallization |

**Table 7**

| **Diffraction angle 2θ** | **d value** | **Intensity %** |
|---|---|---|
| 8.72 | 10.14 | 2.94 |
| 12.49 | 7.09 | 8.24 |
| 12.89 | 6.87 | 28.68 |
| 14.73 | 6.01 | 100.00 |
| 15.76 | 5.62 | 3.36 |
| 16.46 | 5.38 | 2.02 |
| 17.52 | 5.06 | 26.27 |
| 17.93 | 4.95 | 14.40 |
| 18.53 | 4.79 | 1.87 |
| 19.41 | 4.57 | 21.41 |
| 20.19 | 4.40 | 1.59 |
| 20.54 | 4.32 | 2.72 |
| 21.48 | 4.14 | 1.85 |
| 23.25 | 3.83 | 51.50 |
| 24.17 | 3.68 | 2.58 |
| 24.71 | 3.60 | 2.71 |
| 24.91 | 3.58 | 7.24 |
| 25.27 | 3.52 | 17.88 |
| 25.87 | 3.44 | 6.64 |
| 26.44 | 3.37 | 9.40 |
| 26.79 | 3.33 | 4.14 |
| 27.20 | 3.28 | 4.73 |
| 31.37 | 2.85 | 1.28 |
| 31.85 | 2.81 | 2.34 |
| 32.57 | 2.75 | 0.36 |
| 34.06 | 2.63 | 0.72 |
| 35.26 | 2.55 | 0.41 |
| 36.45 | 2.47 | 0.31 |
| 37.44 | 2.40 | 4.42 |
| 39.51 | 2.28 | 2.70 |

### Examples 13-23: Preparation of Crystalline Form A (Anti-Solvent Addition Method)

An appropriate amount of a solid of the compound of formula (I) obtained in Preparation Example 1 was weighed and placed into a 5 mL glass vial at room temperature, and a corresponding volume of a positive solvent was added to produce a clear solution. Then the magnetic stirring (at a rotation speed of about 1000 rpm) was performed to obtain a clear solution, to which a corresponding anti-solvent was added dropwise thereto, and if a solid precipitated, the obtained solid was centrifuged. If no solid precipitated after about 3 mL of the anti-solvent was added, the dropwise addition of the anti-solvent was terminated, the sample was transferred to -20°C and magnetically stirred for about two days to obtain a precipitated solid.

By detection, all of the solids obtained in these examples were Crystalline Form A. The detailed test conditions involved in these examples were shown in Table 8, and the X-ray powder diffraction data of the sample of Example 15 was shown in Table 9.

**Table 8**

| **Example** | **Solid Mass (mg)** | **Positive Solvent** | | **Anti-Solvent** | | **Conditions** |
|---|---|---|---|---|---|---|
| | | **Kind** | **Volume (mL)** | **Kind** | **Volume (mL)** | |
| 13 | 15.1 | Dimethyl sulfoxide | 0.2 | Water | 0.4 | Solid precipitated immediately after adding an anti-solvent dropwise |
| 14 | 15.0 | Methyl | 0.2 | Water | 1.0 | Solid precipitated |
| | | pyrrolidone | | | | immediately after adding an anti-solvent dropwise |
| 15 | 14.5 | Trifluoro ethanol | 0.5 | Methyl tert-butyl ether | 1.8 | Solid precipitated immediately after adding an anti-solvent dropwise |
| 16 | 15.7 | Dimethyl sulfoxide | 0.2 | Methanol | 3.0 | Solid precipitated after stirring at -20°C |
| 17 | 15.6 | Dimethyl formamide | 0.2 | Ethyl acetate | 3.0 | Solid precipitated after stirring at -20°C |
| 18 | 15.3 | Dimethyl formamide | 0.2 | Dichloro methane | 3.0 | Solid precipitated after stirring at -20°C |
| 19 | 14.9 | Dimethyl acetamide | 0.2 | Ethanol | 3.0 | Solid precipitated after stirring at -20°C |
| 20 | 15.8 | Dimethyl acetamide | 0.2 | Toluene | 1.4 | Solid precipitated after stirring at -20°C |
| 21 | 14.3 | Methyl pyrrolidone | 0.2 | Isopropyl acetate | 3.0 | Solid precipitated after stirring at -20°C |
| 22 | 15.1 | Trifluoro ethanol | 0.5 | Acetonitrile | 3.0 | Solid precipitated after stirring at -20°C |
| 23 | 14.6 | Trifluoro ethanol | 0.5 | Acetone | 3.0 | Solid precipitated after stirring at -20°C |

**Table 9**

| **Diffraction angle 2θ** | **d value** | **Intensity %** |
|---|---|---|
| 8.69 | 10.18 | 3.16 |
| 12.51 | 7.08 | 22.79 |
| 12.93 | 6.85 | 41.33 |
| 14.73 | 6.01 | 100.00 |
| 16.52 | 5.37 | 5.17 |
| 17.50 | 5.07 | 21.09 |
| 17.96 | 4.94 | 33.53 |
| 18.52 | 4.79 | 3.49 |
| 19.43 | 4.57 | 37.07 |
| 20.25 | 4.39 | 10.91 |
| 20.60 | 4.31 | 14.38 |
| 21.55 | 4.12 | 3.89 |
| 23.22 | 3.83 | 59.89 |
| 24.18 | 3.68 | 4.34 |
| 24.70 | 3.60 | 6.70 |
| 24.94 | 3.57 | 15.57 |
| 25.27 | 3.52 | 24.34 |
| 25.81 | 3.45 | 5.72 |
| 26.36 | 3.38 | 3.37 |
| 26.76 | 3.33 | 9.28 |
| 27.21 | 3.28 | 3.68 |
| 35.29 | 2.54 | 2.07 |
| 37.47 | 2.40 | 2.83 |

### Examples 24-25: Preparation of Crystalline Form A (Reverse Anti-Solvent Addition Method)

An appropriate amount of a solid of the compound of formula (I) obtained in Preparation Example 1 was weighed and placed into a 3 mL glass vial at room temperature, and a corresponding volume of a positive solvent was added to produce a clear solution. A corresponding anti-solvent was added to a 5 mL glass vial, the magnetic stirring (at a rotation speed of about 1000 rpm) was performed, and then a sample solution was rapidly added. If no solid precipitated after the dropwise addition was completed, the sample was transferred to -20°C and magnetically stirred for about two days to obtain a precipitated solid.

By detection, all of the solids obtained in these examples were Crystalline Form A. The detailed test conditions involved in these examples were shown in Table 10, and the X-ray powder diffraction data of the sample of Example 25 was shown in Table 11.

**Table 10**

| **Example** | **Solid Mass (mg)** | **positive solvent** | | **anti-solvent** | | **Conditions** |
|---|---|---|---|---|---|---|
| | | **Kind** | **Volume (mL)** | **Kind** | **Volume (mL)** | |
| 24 | 14.7 | Dimethylformamide | 0.2 | Water | 3.0 | Solid precipitated immediately after adding an anti-solvent |
| 25 | 15.6 | Methylpyrrolidone | 0.2 | Methyl tert-butyl ether | 3.0 | Solid precipitated after stirring at -20°C |

**Table 11**

| **Diffraction angle 2θ** | **d value** | **Intensity %** |
|---|---|---|
| 8.73 | 10.13 | 5.26 |
| 12.87 | 6.88 | 22.85 |
| 14.73 | 6.01 | 100.00 |
| 17.51 | 5.06 | 58.03 |
| 17.90 | 4.96 | 2.20 |
| 19.37 | 4.58 | 4.59 |
| 20.52 | 4.33 | 1.94 |
| 23.22 | 3.83 | 21.29 |
| 24.15 | 3.69 | 2.60 |
| 24.67 | 3.61 | 3.77 |
| 24.87 | 3.58 | 4.32 |
| 25.24 | 3.53 | 3.42 |
| 26.41 | 3.37 | 14.44 |
| 26.75 | 3.33 | 2.41 |
| 34.66 | 2.59 | 0.40 |
| 37.46 | 2.40 | 0.31 |

### Example 26: Preparation of Crystalline Form B

43.8 mg of a solid of the compound of formula (I) obtained in Preparation Example 1 was weighed and placed into a 1.5 mL glass vial at room temperature, and 0.5 mL of a mixed solvent of toluene/dimethyl sulfoxide (volume ratio 3:1) was added to obtain a suspension. The suspension was magnetically stirred at room temperature (at a rotation speed of about 1000 rpm) for about two weeks, and centrifuged to obtain a solid.

By detection, the solid obtained in this example was Crystalline Form B, its X-ray powder diffraction data was shown in Table 12, the diffraction pattern was shown in Figure 5. This sample had an X-ray powder diffraction pattern comprising characteristic peaks at the 2θ values of about 11.5°±0.2°, about 12.4°±0.2°, about 15.7°±0.2°, about 18.9°±0.2°, about 20.6°±0.2°, about 21.3°±0.2°, about 22.6°±0.2°, about 24.1°±0.2°, about 25.0°±0.2°, and about 27.5°±0.2°.

**Table 12**

| **Diffraction angle 2θ** | **d value** | **Intensity %** |
|---|---|---|
| 8.29 | 10.67 | 5.21 |
| 11.33 | 7.81 | 60.96 |
| 11.55 | 7.66 | 100.00 |
| 12.46 | 7.11 | 33.20 |
| 13.16 | 6.73 | 6.38 |
| 15.46 | 5.73 | 21.83 |
| 15.74 | 5.63 | 63.85 |
| 16.47 | 5.38 | 10.43 |
| 17.33 | 5.12 | 9.26 |
| 18.60 | 4.77 | 25.02 |
| 18.91 | 4.69 | 46.85 |
| 20.67 | 4.30 | 40.40 |
| 21.35 | 4.16 | 49.49 |
| 22.43 | 3.96 | 31.90 |
| 22.62 | 3.93 | 28.43 |
| 22.76 | 3.91 | 21.51 |
| 23.34 | 3.81 | 5.47 |
| 23.69 | 3.76 | 13.72 |
| 24.14 | 3.69 | 23.77 |
| 24.56 | 3.62 | 15.52 |
| 25.07 | 3.55 | 39.57 |
| 25.71 | 3.47 | 5.05 |
| 26.18 | 3.40 | 16.99 |
| 26.51 | 3.36 | 3.44 |
| 27.07 | 3.29 | 3.76 |
| 27.56 | 3.24 | 36.60 |
| 27.92 | 3.20 | 3.89 |
| 28.93 | 3.09 | 12.52 |
| 29.32 | 3.05 | 2.96 |
| 30.08 | 2.97 | 3.51 |
| 30.60 | 2.92 | 2.51 |
| 31.20 | 2.87 | 4.74 |
| 31.77 | 2.82 | 3.63 |
| 34.38 | 2.61 | 4.40 |
| 36.66 | 2.45 | 1.57 |
| 37.37 | 2.41 | 1.51 |
| 37.98 | 2.37 | 8.61 |

### Example 27: Preparation of Crystalline Form C

15.6 mg of a solid of the compound of formula (I) obtained in Preparation Example 1 was weighed and placed into a 3 mL glass vial at room temperature, 1.6 mg of methanol/dichloromethane (volume ratio 1: 1) was added to dissolve the solid, the sample solution was filtered with a polytetrafluoroethylene filter membrane having a pore size of 0.45 microns into a new 3 mL glass vial, which was placed open into a 20 mL glass bottle prefilled with 4 mL of methyl tert-butyl ether. The bottle was sealed, and then placed at room temperature for 12 days to perform the liquid vapor diffusion. After that, the sample was transferred to room temperature and placed open for the volatilization, and separated on the wall to obtain a precipitated solid.

By detection, the solid obtained in this example was Crystalline Form C, its X-ray powder diffraction data was shown in Table 13, the diffraction pattern was shown in Figure 6. This sample had an X-ray powder diffraction pattern comprising characteristic peaks at the 2θ values of about 4.9°±0.2°, about 5.6°±0.2°, about 10.1°±0.2°, about 15.3°±0.2°, about 15.9°±0.2°, about 16.8°±0.2°, about 17.5°±0.2°, about 20.4°±0.2°, about 20.8°±0.2°, about 22.5°±0.2°, and about 26.4°±0.2°. The DSC data for this sample was shown in Figure 7.

**Table 13**

| **Diffraction angle 2θ** | **d value** | **Intensity %** |
|---|---|---|
| 4.87 | 18.15 | 5.06 |
| 5.58 | 15.84 | 100.00 |
| 6.99 | 12.65 | 0.87 |
| 8.76 | 10.10 | 3.82 |
| 9.76 | 9.06 | 2.58 |
| 10.07 | 8.78 | 19.75 |
| 10.36 | 8.54 | 4.04 |
| 11.20 | 7.90 | 3.25 |
| 13.21 | 6.70 | 1.38 |
| 14.05 | 6.31 | 2.24 |
| 14.70 | 6.03 | 0.51 |
| 15.34 | 5.78 | 33.88 |
| 15.92 | 5.57 | 9.13 |
| 16.84 | 5.26 | 10.15 |
| 17.54 | 5.05 | 30.14 |
| 17.84 | 4.97 | 1.97 |
| 20.39 | 4.35 | 26.06 |
| 20.83 | 4.26 | 16.23 |
| 21.16 | 4.20 | 1.99 |
| 21.56 | 4.12 | 1.53 |
| 22.53 | 3.95 | 8.90 |
| 23.70 | 3.76 | 0.98 |
| 25.50 | 3.49 | 0.66 |
| 26.44 | 3.37 | 5.52 |
| 28.28 | 3.16 | 1.13 |

### Example 28: Preparation of Crystalline Form D

15.1 mg of a solid of the compound of formula (I) obtained in Preparation Example 1 was weighed and placed into a 3 mL glass vial at room temperature, and 0.2 mL of dimethyl sulfoxide was added to produce a clear solution. 3 mL of isopropyl acetate was added to a 5 mL glass vial, the magnetic stirring (at a rotation speed of about 1000 rpm) was performed, and then a sample solution was rapidly added thereto, and the sample becomes oil. The sample was transferred to -20°C and the magnetic stirring continued for about three days to obtain a precipitated solid.

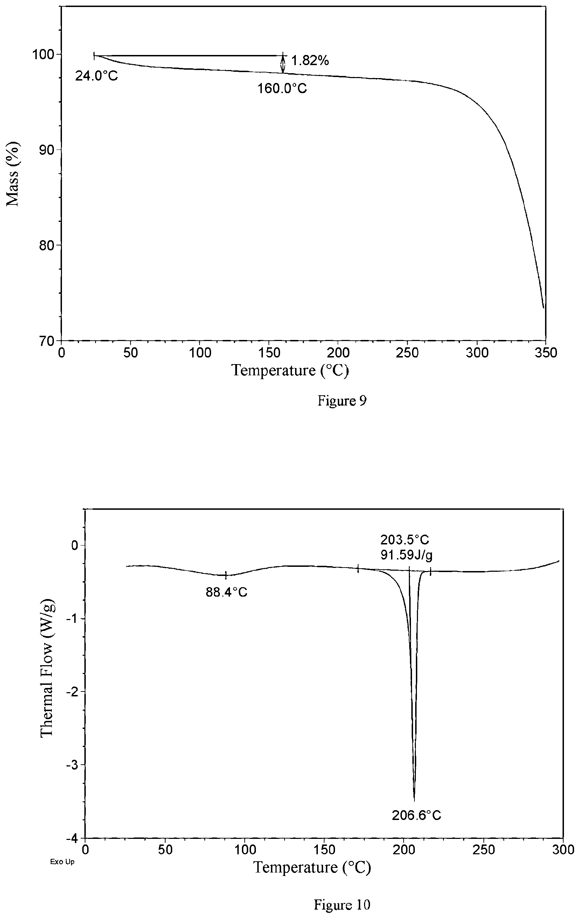

By detection, the solid obtained in this example was Crystalline Form D, its X-ray powder diffraction data was shown in Table 14, the diffraction pattern was shown in Figure 8. This sample had an X-ray powder diffraction pattern comprising characteristic peaks at the 2θ values of about 7.1°±0.2°, about 11.3°±0.2°, about 14.2°±0.2°, about 15.1°±0.2°, about 16.4°±0.2°, about 17.2°±0.2°, about 20.5°±0.2°, about 22.2°±0.2°, about 22.8°±0.2°, about 24.0°±0.2°, and about 24.6°±0.2°. TGA and DSC data for this sample were shown in Figures 9 and 10 respectively.

**Table 14**

| **Diffraction angle 2θ** | **d value** | **Intensity %** |
|---|---|---|
| 7.10 | 12.45 | 100.00 |
| 8.64 | 10.24 | 6.52 |
| 11.07 | 8.00 | 15.13 |
| 11.26 | 7.86 | 23.74 |
| 14.15 | 6.26 | 82.52 |
| 15.13 | 5.86 | 51.04 |
| 15.67 | 5.66 | 10.04 |
| 16.14 | 5.49 | 40.50 |
| 16.43 | 5.39 | 59.93 |
| 16.72 | 5.30 | 15.18 |
| 17.24 | 5.14 | 18.19 |
| 17.63 | 5.03 | 8.50 |
| 18.52 | 4.79 | 4.04 |
| 18.78 | 4.73 | 8.78 |
| 18.94 | 4.69 | 8.09 |
| 19.95 | 4.45 | 15.83 |
| 20.11 | 4.42 | 22.95 |
| 20.49 | 4.34 | 28.15 |
| 21.26 | 4.18 | 4.17 |
| 21.82 | 4.07 | 71.87 |
| 22.16 | 4.01 | 90.86 |
| 22.55 | 3.94 | 23.20 |
| 22.82 | 3.90 | 85.77 |
| 23.12 | 3.85 | 31.90 |
| 23.68 | 3.76 | 20.17 |
| 23.97 | 3.71 | 23.17 |
| 24.67 | 3.61 | 14.60 |
| 26.29 | 3.39 | 2.83 |
| 26.86 | 3.32 | 10.72 |
| 27.20 | 3.28 | 10.61 |
| 28.01 | 3.19 | 11.46 |
| 28.43 | 3.14 | 12.15 |
| 28.91 | 3.09 | 4.94 |
| 29.58 | 3.02 | 15.34 |
| 29.91 | 2.99 | 19.87 |
| 30.33 | 2.95 | 8.00 |
| 31.55 | 2.84 | 2.74 |
| 32.23 | 2.78 | 2.61 |
| 33.13 | 2.70 | 2.93 |
| 34.03 | 2.63 | 7.11 |
| 34.35 | 2.61 | 4.97 |
| 34.71 | 2.58 | 3.70 |
| 35.52 | 2.53 | 16.00 |
| 36.65 | 2.45 | 10.49 |
| 38.08 | 2.36 | 3.68 |
| 39.03 | 2.31 | 4.40 |

### Example 29: Preparation of Crystalline Form E

3-5 mg of a solid of the compound of formula (I) obtained in Preparation Example 1 was weighed and placed into a DSC crucible at room temperature, heated to 210°C at a rate of 10°C/min, and the temperature was maintained for 10 minutes to melt the solid, and then the temperature was reduced to 0°C at a rate of 20°C/min, and the temperature was maintained for 2 minutes to obtain an amorphous solid of the compound of formula (I), which was heated to 150°C at a rate of 10°C/min to recrystallize the sample.

By detection, the solid obtained in this example was Crystalline Form E, its X-ray powder diffraction data was shown in Table 15, the diffraction pattern was shown in Figure 11. This sample had an X-ray powder diffraction pattern comprising characteristic peaks at the 2θ values of about 12.8°±0.2°, about 14.7°±0.2°, about 15.3°±0.2°, about 16.1°±0.2°, about 18.3°±0.2°, about 19.4°±0.2°, about 21.6°±0.2°, about 23.2°±0.2°, about 23.8°±0.2°, about 25.2°±0.2°, and about 26.4°±0.2°. TGA and DSC data were shown in Figures 12 and 13 respectively.

**Table 15**

| **Diffraction angle 2θ** | **d value** | **Intensity %** |
|---|---|---|
| 12.75 | 6.94 | 100.00 |
| 13.11 | 6.75 | 3.81 |
| 14.70 | 6.03 | 8.11 |
| 15.29 | 5.79 | 16.06 |
| 16.10 | 5.50 | 5.73 |
| 16.88 | 5.25 | 1.97 |
| 17.46 | 5.08 | 1.29 |
| 17.84 | 4.97 | 2.67 |
| 18.26 | 4.86 | 13.95 |
| 19.34 | 4.59 | 13.11 |
| 20.48 | 4.34 | 1.31 |
| 21.23 | 4.18 | 7.40 |
| 21.56 | 4.12 | 48.75 |
| 21.88 | 4.06 | 28.42 |
| 23.19 | 3.84 | 6.37 |
| 23.81 | 3.74 | 6.73 |
| 24.36 | 3.65 | 2.64 |
| 25.24 | 3.53 | 3.36 |
| 26.03 | 3.42 | 3.68 |
| 26.42 | 3.37 | 12.39 |
| 26.79 | 3.33 | 8.57 |
| 27.82 | 3.21 | 3.42 |
| 28.74 | 3.11 | 1.37 |
| 30.01 | 2.98 | 1.64 |
| 31.51 | 2.84 | 4.53 |
| 32.54 | 2.75 | 0.49 |
| 34.62 | 2.59 | 2.04 |
| 36.96 | 2.43 | 0.71 |
| 37.42 | 2.40 | 0.49 |
| 38.13 | 2.36 | 1.38 |
| 39.09 | 2.30 | 1.02 |

### Example 30: Preparation of Crystalline Form F

15.2 mg of a solid of the compound of formula (I) obtained in Preparation Example 1 was weighed and placed into a glass vial at room temperature, and 0.4 mL of methanol/dichloromethane (volume ratio 1:1) was added. After filtering, the resulting clear solution was added to another glass vial, which was placed into a 20 mL glass bottle prefilled with 4 mL of methyl tert-butyl ether. After 3 days of the liquid vapor diffusion, a solid precipitated.

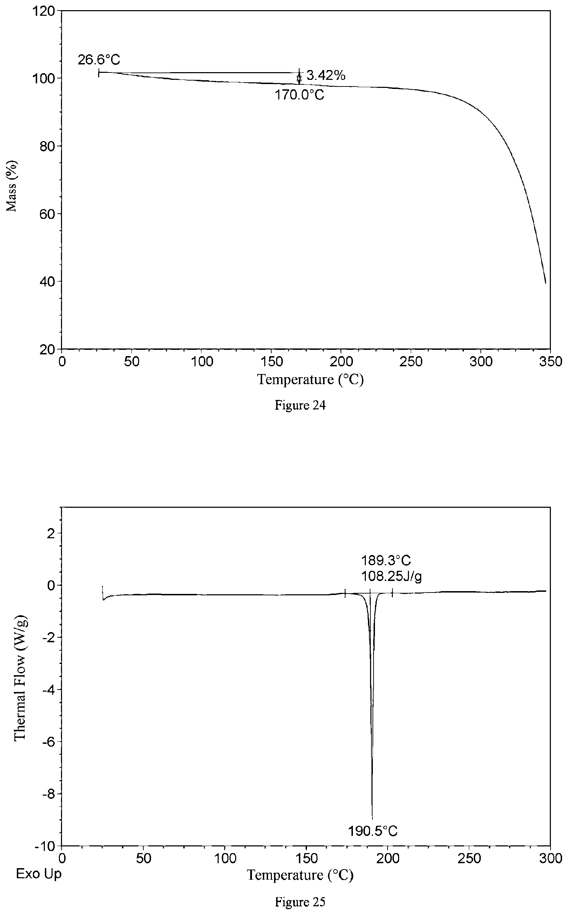

By detection, the solid obtained in this example was Crystalline Form F, its X-ray powder diffraction data was shown in Table 16, the diffraction pattern was shown in Figure 14. This sample had an X-ray powder diffraction pattern comprising characteristic peaks at the 2θ values of about 9.1°±0.2°, about 10.9°±0.2°, about 11.3°±0.2°, about 14.8°±0.2°, about 18.3°±0.2°, about 18.9°±0.2°, about 20.6°±0.2°, about 21.6°±0.2°, about 22.0°±0.2°, about 24.9°±0.2°, and about 25.8°±0.2°. TGA and DSC data were shown in Figures 24 and 25 respectively.

**Table 16**

| **Diffraction angle 2θ** | **d value** | **Intensity %** |
|---|---|---|
| 5.60 | 15.77 | 2.15 |
| 9.09 | 9.73 | 2.42 |
| 10.90 | 8.12 | 100.00 |
| 11.34 | 7.81 | 13.29 |
| 14.34 | 6.18 | 1.94 |
| 14.85 | 5.97 | 22.21 |
| 17.11 | 5.18 | 2.21 |
| 18.35 | 4.83 | 2.47 |
| 19.03 | 4.66 | 7.33 |
| 19.41 | 4.57 | 1.10 |
| 19.76 | 4.49 | 0.90 |
| 20.39 | 4.35 | 0.78 |
| 20.67 | 4.30 | 1.03 |
| 21.31 | 4.17 | 1.41 |
| 21.69 | 4.10 | 0.77 |
| 22.03 | 4.03 | 5.89 |
| 23.70 | 3.75 | 0.80 |
| 24.42 | 3.64 | 1.30 |
| 24.85 | 3.58 | 0.93 |
| 25.07 | 3.55 | 0.68 |
| 25.88 | 3.44 | 1.58 |
| 27.74 | 3.22 | 1.07 |
| 28.22 | 3.16 | 0.34 |
| 28.81 | 3.10 | 0.79 |
| 30.10 | 2.97 | 2.95 |
| 30.58 | 2.92 | 0.61 |
| 34.88 | 2.57 | 0.19 |
| 36.40 | 2.47 | 0.17 |
| 37.00 | 2.43 | 0.57 |
| 37.36 | 2.41 | 0.26 |

### Example 31: Preparation of Crystalline Form F

About 10 mg of a solid of the compound of formula (I) obtained in Preparation Example 1 was weighed and placed into a DSC aluminum crucible, heated to 210°C by DSC at 10°C/min, kept at a constant temperature for 10 minutes, and then cooled to 0°C at 10°C/min. By detection, the solid obtained in this example was Crystalline Form F, its X-ray powder diffraction data was shown in Table 17, the diffraction pattern was shown in Figure 15. This sample had an X-ray powder diffraction pattern comprising characteristic peaks at the 2θ values of about 9.2°±0.2°, about 10.9°±0.2°, about 11.3°±0.2°, about 14.8°±0.2°, about 18.3°±0.2°, about 18.9°±0.2°, about 20.6°±0.2°, about 21.6°±0.2°, about 22.0°±0.2°, about 24.9°±0.2°, and about 25.8°±0.2°.

**Table 17**

| **Diffraction angle 2θ** | **d value** | **Intensity %** |
|---|---|---|
| 9.16 | 9.65 | 0.81 |
| 10.90 | 8.12 | 100.00 |
| 11.34 | 7.80 | 3.55 |
| 14.77 | 6.00 | 19.81 |
| 18.48 | 4.80 | 1.64 |
| 18.87 | 4.70 | 2.15 |
| 19.23 | 4.62 | 3.89 |
| 20.61 | 4.31 | 2.98 |
| 21.57 | 4.12 | 1.53 |
| 21.97 | 4.05 | 6.74 |
| 23.63 | 3.76 | 1.12 |
| 24.88 | 3.58 | 2.48 |
| 25.82 | 3.45 | 1.98 |
| 29.84 | 2.99 | 2.19 |
| 34.55 | 2.60 | 0.16 |
| 36.71 | 2.45 | 0.36 |

It should be noted that, affected by the preferred orientation of samples, sample preparation methods, instrument testing, and data processing errors, the relative intensities of XRPD diffraction peaks of different samples of the same crystalline form may be different, and the positions of diffraction peaks may be different. Specifically, due to factors such as the preferred orientation of samples, sample preparation methods, and testing methods, the relative order of the diffraction peak intensities may be different in different samples of the same crystalline form; due to the influence of instrument testing and data processing errors, there may be deviations in the positions of the diffraction peaks of different batches of samples of the same crystalline form, the general deviation range is within ±0.2°, and the interference of test and data processing on the peak positions is more obvious for the diffraction peaks with weaker intensity. By combining the above factors, in the determination of the crystalline form, the diffraction peaks of the Crystalline Form F obtained in examples 30 and 31 were compared based on the peak positions of the main diffraction peaks of the sample, wherein the main diffraction peaks (peak intensity >5%) of the sample of one example could correspond to the main diffraction peaks of the sample of the other example, which indicated that the two samples were substantially the same crystalline form.

### Example 32: Solubility of Crystalline Forms

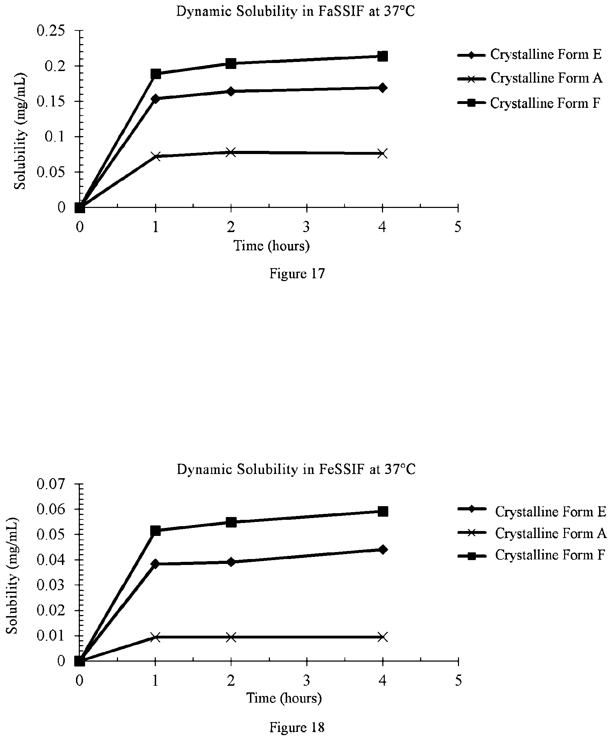

Crystalline Form E of the present invention (prepared in multiple batches in parallel by the process of Example 29), Crystalline Form F of the present invention (prepared in multiple batches in parallel by the process of Example 31) and Crystalline Form A of WO2020/008391A were formulated into suspensions with FaSSIF (Fasted State Simulated Intestinal Fluid), FeSSIF (Fed State Simulated Intestinal Fluid) and pure water , equilibrated at 37°C for 1 hour, 2 hours, and 4 hours, and then filtered to obtain saturated solutions. The sample contents in saturated solutions were determined by high performance liquid chromatography (HPLC). The experimental results were shown in Table 18, and the solubility curves were shown in Figures 16-18, respectively. The experimental results showed that the solubility of Crystalline Form E of the present invention in FaSSIF, FeSSIF and pure water were higher than that of Crystalline Form A of the prior art.

**Table 18**

| Biological Media | **Time** | WO2020/008391A Crystalline Form A | **Crystalline Form E of the present invention** | **Crystalline Form F of the present invention** |
|---|---|---|---|---|
| Solubility in water (mg/mL) | 1 hour | 0.0643 | 0.1147 | 0.1796 |
| | 2 hours | 0.0747 | 0.1386 | 0.1892 |
| | 4 hours | 0.0723 | 0.1562 | 0.2030 |
| Solubility in FeSSIF (mg/mL) | 1 hour | 0.0094 | 0.0384 | 0.0516 |
| | 2 hours | 0.0094 | 0.0392 | 0.0549 |
| | 4 hours | 0.0096 | 0.0441 | 0.0592 |
| Solubility in FaSSIF (mg/mL) | 1 hour | 0.0723 | 0.1538 | 0.1890 |
| | 2 hours | 0.0780 | 0.1645 | 0.2035 |
| | 4 hours | 0.0761 | 0.1694 | 0.2142 |

### Example 33: Compressibility of Crystalline Forms

A manual tableting machine was used to perform the tableting. Upon tableting, a circular flat punch that allows to suppress into cylindrical tablets was selected, and certain amounts of Crystalline Form E obtained in Example 29 of the present invention and Crystalline Form A of WO2020/008391A are added respectively. 10 KN pressure was applied to suppress into cylindrical tablets, which were placed in a dryer for 24 hours. After the complete elastic restoration, the tablets were tested with a tablet hardness meter for its radial crushing force (hardness, H). Diameter (D) and thickness (L) of the tablets were measured with a Vernier caliper, and the tensile strength of the powder under different hardness was calculated using the formula T=2H/πDL. The experimental results were shown in Table 19. Under a certain pressure, the higher the tensile strength was, the better the compressibility was. Crystalline Form E of the present invention had higher tensile strength and better compressibility than Crystalline Form A of the prior art.

**Table 19**

| Crystalline Form | Tablet Diameter (mm) | Tablet thickness (mm) | hardness (N) | Tensile strength (MPa) |
|---|---|---|---|---|
| A | 6 | 1 | 17.01 | 1.805 |
| E | 6 | 0.7 | 17.20 | 2.607 |

### Example 34: Comparative Study on Stability

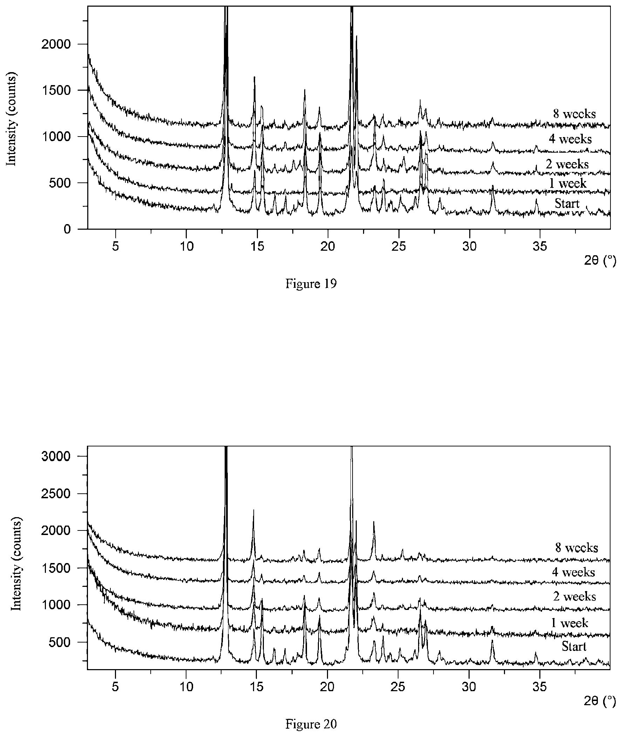

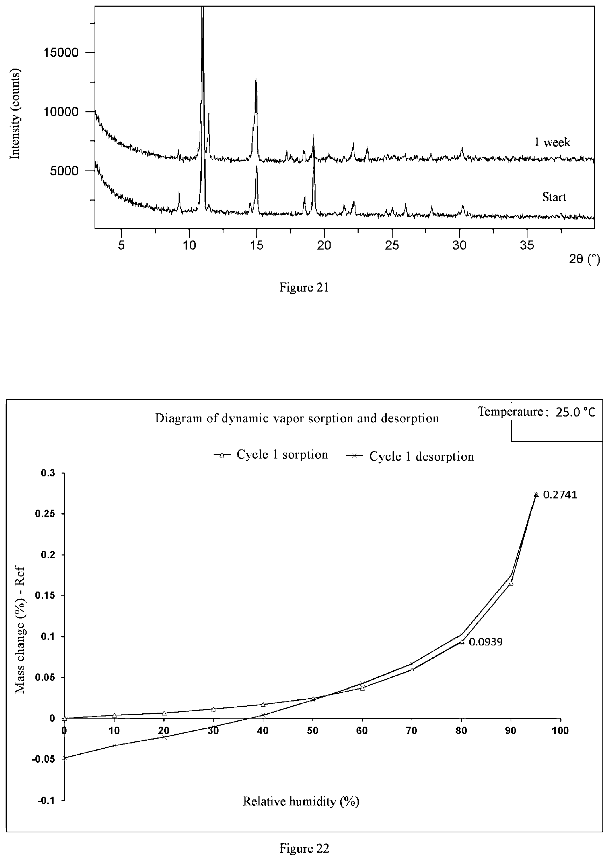

About 15 mg of Crystalline Form E (initial purity 99.42%) obtained in Example 29 of the present invention was weighed, and placed open in a stabilization box under the conditions of 25°C/60%RH and 40°C/75%RH. After 1 week, 4 weeks and 8 weeks, samples were taken to measure XRPD and HPLC, and about 2 mg of Crystalline Form F (initial purity 98.09%) obtained in Example 30 of the present invention was weighed, and placed in a stabilization box under the condition of 40°C/75%RH. After 1 week, samples were taken to measure XRPD and HPLC. The experimental results were shown in Table 20. The stability of Crystalline Form E was shown in Figure 19 and Figure 20, and the stability of Crystalline Form F was shown in Figure 21. The crystalline form and the purity did not change significantly after being placed, and Crystalline Forms E and F of the present invention had relatively good physical/chemical stability under the conditions of 25°C/60%RH and 40°C/75%RH.

**Table 20**

| Crystalline Form | Open (Yes/No) | Temperature/Humidity conditions | Time | Solid form | HPLC Relative Purity * |
|---|---|---|---|---|---|
| Crystalline Form E | Yes | 25°C, 60%RH | 1 week | Crystalline Form E | 100.2% |
| | Yes | 40°C, 75%RH | | Crystalline Form E | 100.2% |
| | Yes | 25°C, 60%RH | 2 weeks | Crystalline Form E | 100.0% |
| | Yes | 40°C, 75%RH | | Crystalline Form E | 100.4% |
| | Yes | 25°C, 60%RH | 4 weeks | Crystalline Form E | 100.0% |
| | Yes | 40°C, 75%RH | | Crystalline Form E | 100.4% |
| | Yes | 25°C, 60%RH | 8 weeks | Crystalline Form E | 100.1% |
| | Yes | 40°C, 75%RH | | Crystalline Form E | 100.2% |
| Crystalline Form F | Yes | 40°C, 75%RH | 1 week | Crystalline Form F | 99.09% |

| | | | | | |
|---|---|---|---|---|---|
| ^{∗} Relative purity = HPLC purity of test sample/HPLC purity of starting sample× 100% | | | | | |

### Example 35: Comparative Study on Hygroscopicity

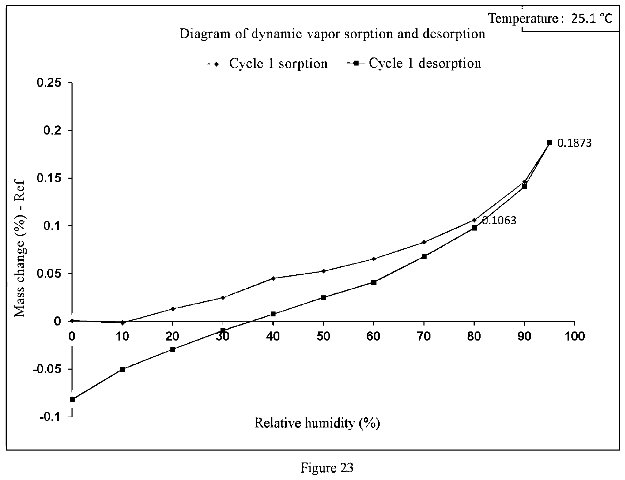

Each about 10 mg of Crystalline Form of the present invention and Crystalline Form A of WO2020/008391A was weighed for dynamic vapor sorption (DVS) test, and then sampled to measure XRPD. The experimental results were shown in Table 21, and the dynamic vapor sorption diagrams of Crystalline Forms A and E were shown in Figures 22-23 respectively. The hygroscopic properties of Crystalline Form E of the present invention and Crystalline Form A of the prior art were comparable at 80% relative humidity, but Crystalline Form E of the present invention had lower hygroscopicity compared with Crystalline Form A of the prior art at 95% relative humidity.

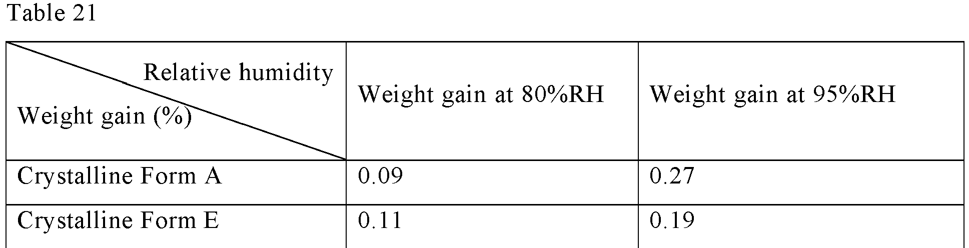

The description about hygroscopicity characteristics and the definition of the weight gain due to hygroscopicity (Chinese Pharmacopoeia 2010 Edition Appendix XIX J Guidelines for the hygroscopicity test of drugs):
Deliquescence: Sufficient water was absorbed to form a liquid.
Very hygroscopic: The weight gain due to hygroscopicity was not less than 15%
Hygroscopic: The weight gain due to hygroscopicity was less than 15% but not less than 2%
Slightly hygroscopic: The weight gain due to hygroscopicity was less than 2% but not less than 0.2%
Not or nearly not hygroscopic: The weight gain due to hygroscopicity was less than 0.2%

### Example 36: Intrinsic Dissolution Rate of Crystalline Form

Each about 100 mg of Crystalline Form E obtained in Example 29 of the present invention and Crystalline Form A of WO2020/008391A were weighed, poured into the intrinsic dissolution mold, and maintained under a pressure of 5 kN for 1 minute to prepare a sheet with a surface area of 0.5 cm². An intact pressed sheet was transferred to the dissolution apparatus for testing the intrinsic dissolution rate, and the slope was calculated according to the measurement points between 10 and 30 minutes, expressed in mg/mL, and the intrinsic dissolution rate (IDR) was further calculated according to the slope, expressed in mg/min/cm². The dissolution rate of Crystalline Form E of the present invention was faster than that of Crystalline Form A of the prior art.

### Example 37: Comparative Study on Crystal Habit

Each about 10 mg of Crystalline Forms of the present invention and Crystalline Form A of WO2020/008391A were weighed, and placed on glass slides respectively. A little vacuum silicone oil was added dropwise to disperse the sample. Then the sample was covered with a cover slip, and placed under a polarizing microscope for observation. Compared with Crystalline Form A of the prior art, Crystalline Forms of the present invention had a better crystal habit.

### Example 38: Comparative Study on Particle Size Distribution

Each about 10-30 mg of Crystalline Forms of the present invention and Crystalline Form A of WO2020/008391A was weighed, and then about 5 mL of Isopar G (containing 0.2% lecithin) was added. The samples to be tested were intensively mixed to become uniform, and then added to the SDC sampling system. When the shading degree reached an appropriate range, the experiment was started, and the particle size distribution test was performed after the ultrasonic treatment for 30 seconds. Compared with Crystalline Form A of the prior art, Crystalline Forms of the present invention had a more uniform particle size distribution.

### Example 39: Comparative Study on Adhesion

Each about 30 mg of Crystalline Forms of the present invention and Crystalline Form A of WO2020/008391A were weighed, and then added into an 8 mm circular flat punch. A tableting treatment was performed with a pressure of 10 kN. After tableting, the punch was maintained for about half a minute, and then the amount of powder adsorbed on the punch was weighed. After two consecutive pressings using this method, the final adhesion amount accumulated on the punch, and the highest adhesion amount and the average adhesion amount during the pressing process were recorded. The adhesion of Crystalline Forms of the present invention was better than that of Crystalline Form A of the prior art. The above-mentioned embodiments are only intended to illustrate the technical concept and characteristics of the present invention, and the purpose thereof is to enable those who are familiar with the art to understand the content of the present invention and implement them accordingly, and cannot limit the protection scope of the present invention. All equivalent changes or modifications made according to the spirit of the present invention should be included within the protection scope of the present invention.

## Claims

1. E-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form E, which is **characterized in that**, by using the Cu-Kα radiation, Crystalline Form E has an X-ray powder diffraction pattern comprising characteristic peaks at the 2θ values of 12.8°±0.2°, 15.3°±0.2°, and 21.6°±0.2°,

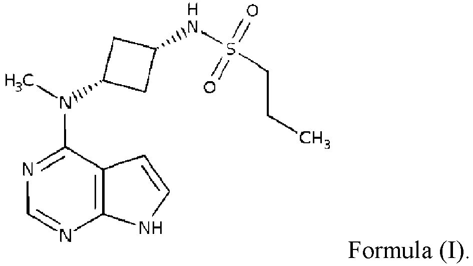

2. A process for preparing Crystalline Form E of claim 1, which is **characterized in that** said process comprises:
step (1): heating the compound of formula (I) to 200°C-220°C,
step (2): then cooling to 0°C-5°C,
step (3): reheating to 140°C-160°C to obtain Crystalline Form E.

3. F-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form F, which is **characterized in that**, by using the Cu-Kα radiation, Crystalline Form F has an X-ray powder diffraction pattern comprising characteristic peaks at the 2θ values of 10.9°±0.2°, 14.8°±0.2°, and 20.6°±0.2°,

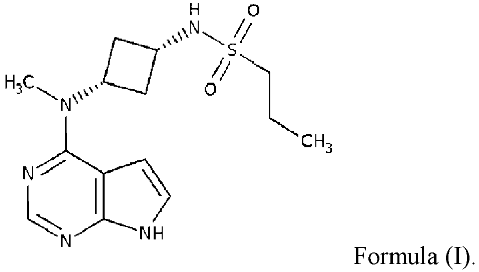

4. A process for preparing Crystalline Form F of claim 3, which is **characterized by** a solid of the compound of formula (I) is heated, and then cooled to obtain Crystalline Form F.

5. C-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form C, which is **characterized in that**, by using the Cu-Kα radiation, Crystalline Form C has an X-ray powder diffraction pattern comprising characteristic peaks at the 2θ values of 5.6°±0.2°, 10.1°±0.2°, and 15.3°±0.2°,

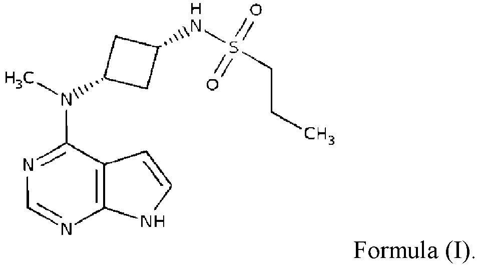

6. A process for preparing Crystalline Form C of claim 5, which is **characterized by** the compound of formula (I) is dissolved in methanol/dichloromethane, the resulting system is placed under an atmosphere of methyl tert-butyl ether to perform the liquid vapor diffusion, then volatilized, and separated to obtain Crystalline Form C.

7. D-type crystal of a compound represented by formula (I) N-(((1 S,3 S)-3-(methyl(7H-pyrrolo[2,3-d]pyrimidin-4-yl)amino)cyclobutyl)propane-1-sulf onamide, i.e., Crystalline Form D, which is **characterized in that**, by using the Cu-Kα radiation, Crystalline Form D has an X-ray powder diffraction pattern comprising characteristic peaks at the 2θ values of 7.1°±0.2°, 14.2°±0.2°, and 15.1°±0.2°,

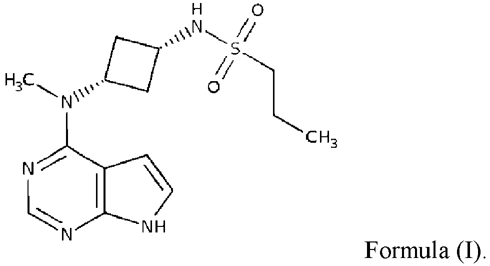

8. A process for preparing Crystalline Form D of claim 7, which is **characterized by** the compound of formula (I) is dissolved in dimethyl sulfoxide, added to isopropyl acetate, and then transferred to a temperature between -25°C and -10°C to obtain Crystalline Form D.

9. B-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form B, which is **characterized in that**, by using the Cu-Kα radiation, Crystalline Form B has an X-ray powder diffraction pattern comprising characteristic peaks at the 2θ values of 11.5°±0.2°, 15.7°±0.2°, and 18.9°±0.2°,

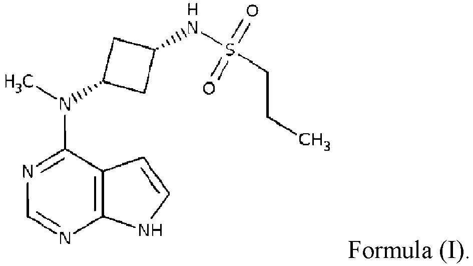

10. A process for preparing Crystalline Form B of claim 9, which is **characterized by** the compound of formula (I) is added to toluene/dimethyl sulfoxide, and the suspension system is stirred to obtain Crystalline Form B.

11. A process for preparing A-type crystal of a compound represented by formula (I) N-(((1 S,3 S)-3-(methyl(7H-pyrrolo[2,3-d]pyrimidin-4-yl)amino)cyclobutyl)propane-1-sulf onamide, i.e., Crystalline Form A, which is **characterized in that**, 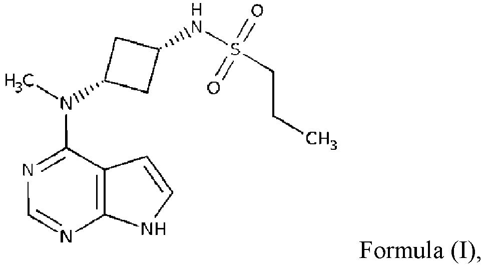 the compound of formula (I) is dissolved in a positive solvent, and after filtering, an anti-solvent is dropwise added thereto to obtain Crystalline Form A.

12. A process for preparing A-type crystal of a compound represented by formula (I) N-(((1S,3 S)-3-(methyl(7H-pyrrolo[2,3-d]pyrimidin-4-yl)amino)cyclobutyl)propane-1-sulf onamide, i.e., Crystalline Form A, which is **characterized in that**, 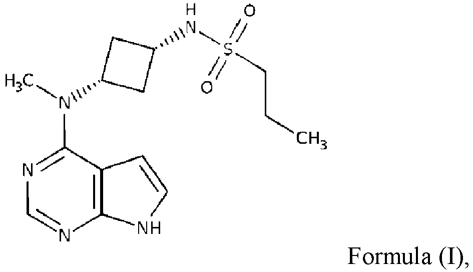 the compound of formula (I) is dissolved in a positive solvent, and after filtering, quickly added to an anti-solvent to obtain Crystalline Form A.

13. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is **characterized in that**, 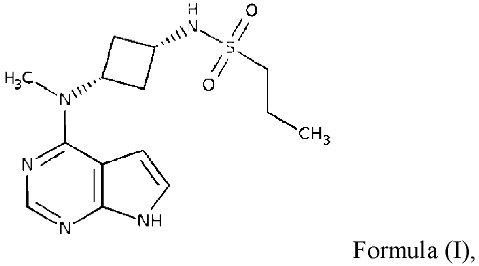 the compound of formula (I) is dissolved in at least one of methanol, tetrahydrofuran, 1,4-dioxane, trifluoroethanol, acetic acid, acetonitrile/pure water, methanol/dichloromethane, 40% aqueous dimethylamine solution and tetrahydrofuran/water, and after filtering, the solvent is volatilized until a solid precipitates to obtain Crystalline Form A.

14. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is **characterized in that**, 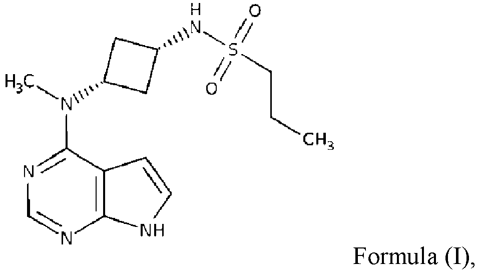 the compound of formula (I) is dissolved in an organic solvent or a mixed solvent system, filtered after reaching a dissolution equilibrium under a high temperature condition, and slowly cooled until a solid precipitates to obtain Crystalline Form A.

15. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is **characterized in that**, 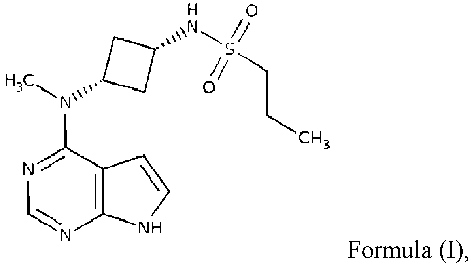 an excessive amount of the compound of formula (I) is added to an organic solvent or a mixed solvent system, and the suspension system is stirred to obtain Crystalline Form A.

16. A process for preparing A-type crystal of a compound represented by formula (I) N-(((1S,3S)-3-(methyl(7H-pyrrolo[2,3-d]pyrimidin-4-yl)amino)cyclobutyl)propane-1-sulf onamide, i.e., Crystalline Form A, which is **characterized in that**, 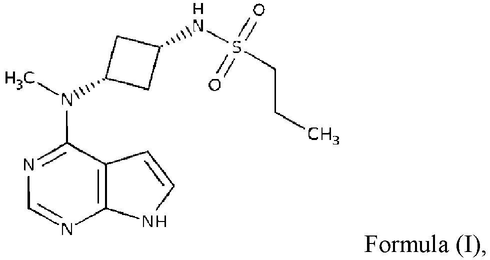 the compound of formula (I) is dissolved in a positive solvent, and placed under an anti-solvent atmosphere to perform the liquid vapor diffusion, and then the resulting system is volatilized and separated to obtain Crystalline Form A.

17. A pharmaceutical composition, containing the crystal according to any one of claims 1, 3, 5, 7 and 9 and a pharmaceutically acceptable carrier.

18. A pharmaceutical composition having JAK1 inhibitory activity, containing the crystal according to any one of claims 1, 3, 5, 7 and 9 as an active component.

19. A preventing or treating drug for atopic dermatitis, lupus vulgavis and/or plaque psoriasis, which contains the crystal according to any one of claims 1, 3, 5, 7 and 9 as an active component.

20. A process for preparing Crystalline Form F according to claim 3, which is **characterized in that** the compound of formula (I) is added to methanol/dichloromethane, and after filtering, the filtrate is subjected to the liquid vapor diffusion with methyl tert-butyl ether to obtain Crystalline Form F.
